(19) Europäisches Patentamt
European Patent Office
Office européen des brevets

(11) EP 2 137 209 B1

(12) **FASCICULE DE BREVET EUROPEEN**

(45) Date de publication et mention de la délivrance du brevet:
**09.02.2011 Bulletin 2011/06**

(21) Numéro de dépôt: **08718333.1**

(22) Date de dépôt: **28.03.2008**

(51) Int Cl.:
*C07K 7/06 (2006.01)* *A61K 38/08 (2006.01)*
*G01N 33/68 (2006.01)*

(86) Numéro de dépôt international:
**PCT/EP2008/053768**

(87) Numéro de publication internationale:
**WO 2008/125464 (23.10.2008 Gazette 2008/43)**

(54) **COMPOSES POUR LE DIAGNOSTIQUE DE MALADIES LIEES A L'EXPRESSION DE VCAM**

VERBINDUNGEN ZUR DIAGNOSE VON MIT DER VCAM-EXPRESSION ASSOZIIERTEN KRANKHEITEN

COMPOUNDS FOR THE DIAGNOSIS OF DISEASES ASSOCIATED WITH VCAM EXPRESSION

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **28.03.2007 FR 0754087**

(43) Date de publication de la demande:
**30.12.2009 Bulletin 2009/53**

(60) Demande divisionnaire:
**10195948.4**

(73) Titulaire: **GUERBET**
**93420 Villepinte (FR)**

(72) Inventeurs:
- **PORT, Marc**
  **F-95170 Deuil La Barre (FR)**
- **ROUSSEAUX, Olivier**
  **F-60300 Senlis (FR)**
- **MULLER, Robert**
  **B-7000 Mons (BE)**
- **BURTEA, Carmen**
  **B-7000 Mons (BE)**

(74) Mandataire: **Warcoin, Jacques et al**
**Cabinet Régimbeau**
**20, rue de Chazelles**
**75847 Paris Cedex 17 (FR)**

(56) Documents cités:
**WO-A-03/038130** **WO-A-2004/112840**
**FR-A- 2 876 033** **US-A- 6 110 457**
**US-A1- 2002 048 763** **US-A1- 2003 077 288**

- **KELLY KA ET AL: "Detection of Vascular Adhesion Molecule-1 Expression Using a Novel Multimodal Nanoparticle" CIRCULATION RESEARCH, vol. 96, 18 février 2005 (2005-02-18), pages 327-336, XP002456858**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 137 209 B1

## Description

L'invention concerne de nouveaux composés de diagnostique de maladies liées à l'expression de VCAM, leur procédé de préparation et leur utilisation en imagerie médicale.

VCAM-1 (vascular cell adhesion molecule - 1) est une immunoglobuline d'intérêt notamment diagnostique dans les pathologies cardio-vasculaires. En effet, VCAM-1 est un marqueur des zones prédisposées à devenir athéromateuses. VCAM-1 est fortement surexprimée sur les cellules endothéliales activées. Comme d'autres molécules d'adhésion, telles que ICAM-1, 2, et 3, VCAM-1 est impliquée dans l'adhésion des leucocytes à l'endothélium au cours de l'athérosclérose. Plusieurs modèles in vivo ont été décrits. Dans les lésions athérosclèreuses avancées, l'expression de VCAM-1 est positive au niveau de l'endothélium coiffant la plaque ; VCAM-1 est également présente sur les cellules endothéliales à proximité des lésions et elle est très abondante dans l'intima, au niveau des cellules musculaires lisses.

Chez l'homme, l'expression de VCAM-1 dans les artères athérosclèreuses aortiques et fémorales humaines a été décrite de la façon suivante :

artère d'apparence saine : faible expression au niveau de l'endothélium artère avec épaississement du néo-intima, sans infiltration macrophagique : légère augmentation endothéliale en VCAM-1
lésion développée, contenant des macrophages « matures » : expression doublée par rapport à la situation précédente
lésion développée, comportant une infiltration récente en macrophages : immunomarquage significativement plus élevé, recouvrant une part élevée de la zone endothéliale.

Au niveau des coronaires VCAM1 est exprimée seulement sur l'endothélium des plaques, et non au niveau des parois saines.

Dans le domaine oncologie, VCAM participe à l'adhésion des lymphocytes, des monocytes et des polynucléaires éosinophiles sur les cellules endothéliales activées via l'intégrine alpha4-betal. Elle est fortement exprimée dans des pathologies telles que la colite ulcérative chronique ou la maladie de Crohn. Comme ICAM-1, elle serait surexprimée à la périphérie des cancers et pourrait participer aux processus métastatiques.

On recherche toujours une amélioration de la qualité du diagnostic in vivo de maladies cardiovasculaires et cancéreuses à l'aide de nouveaux marqueurs très spécifiques, destinés aux modalités d'imagerie connues de l'homme du métier, notamment l'IRM, les rayons X, la scintigraphie aux rayons gamma, le scanner CT, les ultrasons, le PET, l'imagerie optique. On rappelle que dans le cas de l'IRM, un contraste est obtenu grâce à l'administration d'agents de contraste contenant des métaux paramagnétiques ou superparamagnétiques qui ont un effet sur la relaxivité des protons de l'eau. Dans le cas de la scintigraphie, le contraste est obtenu par la localisation spécifique d'un composé radiopharmaceutique émettant des rayons gamma ou beta.

On cherche des composés dont la synthèse chimique n'est pas trop complexe, suffisamment stables in vivo pour une utilisation en imagerie médicale, et dont le prix de revient n'est pas trop élevé, et cela notamment pour l'IRM afin de ne pas utiliser d'éléments radioactifs complexes d'utilisation.

Le demandeur a réussi à obtenir des composés comprenant une partie de ciblage de zones VCAM, efficaces non seulement in vitro mais aussi et surtout en IRM in vivo. De tels composés sont en effet difficiles à obtenir car il faut non seulement identifier un biovecteur efficace in vitro, mais obtenir un composé efficace en imagerie de diagnostique clinique humaine. C'est particulièrement le cas pour l'IRM qui est reconnue pour être une technique très demandée car sans radioactivité, mais dont la sensibilité est très nettement inférieure à celle de la médecine nucléaire.

Le composé obtenu doit à la fois avoir une affinité suffisante pour reconnaître sa cible, une forte spécificité pour être un indicateur distinctif de l'état pathologique, une stabilité appropriée pour ne pas être dégradé ou modifié in vivo ; et en plus sans que la partie de signal n'interfère de manière gênante sur ces différents paramètres (affinité et stabilité en particulier).

Après de nombreuses tentatives, le demandeur a réussi à obtenir des composés efficaces.

L'invention concerne ainsi un composé de formule générale (I) suivante :

Signal - Lien - Peptide (I)
dans laquelle :

- Signal représente une entité signal ;
- Lien, présent ou non, représente une liaison chimique et
- Peptide représente un peptide ciblant VCAM, consistant au plus en 20 aminoacides le peptide étant choisi parmi les peptides de formule suivante:

  a) X1-X2-X3-X4-X5-X6-X7-X8-X9 (1) (SEQ ID No1) avec :

- X1 est absent ou choisi parmi la cystéine, la méthionine
- X2 choisi parmi l'asparagine ou la glutamine
- X3 choisi parmi l'asparagine ou la glutamine
- X4 choisi parmi la sérine, la thréonine
- X5 choisi parmi la lysine, l'arginine, l'histidine, l'ornithine
- X6 choisi parmi la sérine, la thréonine
- X7 choisi parmi l'histidine, l'arginine, la lysine
- X8 choisi parmi la thréonine, la sérine
- X9 est absent ou choisi parmi la cystéine, la méthionine de préférence le peptide CNNSKSHTC (SEQ ID No2) (Cys-Asn-Asn-Ser-Lys-Ser-His-Thr-Cys)

et le peptide NNSKSHT (SEQ ID No3)
et les sels pharmaceutiquement acceptables de ces composés du a).

L'expression « peptide ciblant VCAM » est également désignée PEPTIDE VCAM dans la demande. Avantageusement Peptide représente un PEPTIDE VCAM.
Le PEPTIDE VCAM selon l'invention consiste au plus en 20 aminoacides, avantageusement au plus en 15 aminoacides, avantageusement au plus en 10 aminoacides.
La protéine KIAA0137 et de la protéine HPIV-3 sont exclues des PEPTIDES VCAM, en particulier lorsque Signal représente un marqueur pour l'imagerie optique telle qu'une molécule fluorescente. La protéine KIAA0137 est en particulier décrite dans la demande de brevet WO 03/038130 et la protéine HPIV-3 est en particulier décrite dans le brevet US 6 110 457.
Par l'expression peptide CNNSKSHTC (SEQ ID No2), NNSKSHT (SEQ ID No3) et leurs équivalents fonctionnels, on entend le peptide CNNSKSHTC(SEQ ID No2), le peptide NNSKSHT (SEQ ID No3) dont le demandeur a démontré l'efficacité, et les peptides de formule X1-X2-X3-X4-X5-X6-X7-X8-X9 (1) (SEQ ID No1) dérivés qui une fois inclus dans le composé (I) présentent une efficacité en imagerie semblable ou meilleure que le CNNSKSHTC (SEQ ID No2) (ce qui inclut les peptido-mimétiques), cette efficacité étant testée à l'aide de tests et de modèles in vivo décrits en détail dans la demande ou de modèles analogues appropriés. Le peptide CNNSKSHTC (SEQ ID No2) étudié est un peptide cyclique et est exemplifié en détail dans la demande.
En particulier, un dérivé du peptide CNNSKSHTC (SEQ ID No2) et NNSKSHT (SEQ ID No3) inclut un peptide ou composé dans lequel la séquence NNSKSHT (SEQ ID No3) constituant la séquence a été modifiée par ajout, suppression, substitution ou modification d'au moins un acide aminé.
La substitution peut être conservative ou non. La substitution est conservative lorsqu'un acide aminé est substitué par un acide aminé ayant des propriétés similaires (par exemple, polarité, potentiel de liaison d'hydrogène, acidité, basicité, hydrophobicité, présence d'un groupe aromatique, etc). Un acide aminé naturel peut être remplacé par un acide aminé non naturel, tel qu'un acide aminé en configuration D, acide aminé bêta ou gamma. Le PEPTIDE VCAM est par exemple modifié en utilisant des méthodologies appropriées décrites dans l'art antérieur par exemple dans US2005100963 (colonne 20-21 paragraphes [529] à [541] dans le cas de peptides ciblant des récepteurs KDR), afin de sélectionner des composés (I) efficaces.
Dans le cas de CNNSKSHTC (SEQ ID No2) et NNSKSHT (SEQ ID No3)

- N l'asparagine peut être remplacé par un autre acide aminé porteur d'une chaîne amide notamment la glutamine, ou des dérivés substitués par exemple alkyl substitués
- S la sérine peut être remplacé par un autre acide aminé porteur d'un groupe hydroxy, par exemple la thréonine, l'homosérine
- K la lysine peut être remplacé par d'autres acides aminés dibasiques (arginine, histidine, ornithine) ou des dérivés de la lysine ou de ces autres acides aminés, par exemple substitués par des alkyles C1 à C10
- H l'histidine peut être remplacé par d'autres acides aminés hydrophiles basiques notamment l'arginine, la lysine
- T la thréonine peut être remplacé par la sérine

La séquence NNSKSHT (SEQ ID No3) peut être modifiée en remplaçant une ou plusieurs liaisons amide par une liaison conférant une stabilité accrue in vivo, par exemple conférant une résistance accrue à la protéolyse.
On entend par peptide comprenant au moins un peptide ciblant VCAM, un peptide présentant cette séquence peptidique de reconnaissance de la cible biologique, éventuellement flanquée en extrémité N et/ou C terminal d'un groupe chimique n'interférant pas sur l'efficacité en imagerie de cette séquence.
On entend par le terme Signal ou entité signal une entité chimique permettant d'obtenir un signal en imagerie médicale, en particulier :

- un chélate capable d'être couplé à un métal paramagnétique
- une nanoparticule métallique, notamment une nanoparticule superparamagnétique d'oxyde de fer
- une nanoparticule lipidique, avantageusement sous forme d'émulsion, cette nanoparticule étant porteuse d'au moins un chélate capable d'être couplé à un métal paramagnétique (dans ce cas, le Peptide est greffé à la nanoparticule lipidique en émulsion qui est elle-même porteuse de chélates ; la liaison du peptide à la nanoparticule lipidique se fait par exemple par un groupe de liaison chimique).

Selon un mode de réalisation, l'entité signal comprend au moins un chélate Ch (sous forme complexant un métal M). Avantageusement le chélate est couplé au métal M. Avantageusement, le métal M est un ion de métal paramagnétique, ou un radionucléide. Le complexe formé par le chélate et le métal M est stable dans les conditions physiologiques de manière à éviter une libération non souhaitée du métal M dans l'organisme. Avantageusement le chélate ou l'entité signal comprend au moins un groupe fonctionnel permettant de relier l'entité signal avec le Lien ou directement avec le Peptide. L'invention concerne aussi les composés de formule (I) dans lesquels le chélate n'est pas complexé avec le métal.

Avantageusement, Ch est un chélate linéaire choisi parmi : EDTA, DTPA diéthylènetriaminopentaacétique acide, N-[2-[bis(carboxyméthyl)amino]-3-(4-ethoxyphenyl)propyl]-N-[2-[bis(carboxyméthyl)amino]éthyle]-L-glycine (EOB-DTPA), , des dérivés mono- ou bis-amide du DTPA, tels que N,N-bis[2-[carboxyméthyl[(méthylcarbamoyl)méthyle] amino]éthyle] glycine (DTPA-BMA), 4-carboxy-5,8,11-tris(carboxyméthyl)-1-phenyl-2-oxa-5,8,11-triazatridecan-13-oic acide (BOPTA).

De façon avantageuse, Ch est un chélate macrocyclique choisi parmi 1,4,7,10-tetraazacyclododecan-1,4,7,10-tetraacetic acide (DOTA), 1,4,7,10-tetraazacyclododecan-1,4,7-triacetic acide (DO3A), 10-(2-hydroxypropyl)-1,4,7,10-tetraazacyclododecan-1,4,7-triacetic acide (HPDO3A) 2-methyl-1,4,7,10-tetraazacyclododecan-1,4,7,10-tetraacetic acide (MCTA), (alpha , alpha ', alpha ", alpha"')-tetramethyl-1,4,7,10-tetraazacyclododecan-1,4,7,10-tetraacetic acide (DOTMA), 3,6,9,15-tetraazabicyclo[9.3.1]pentadeca-1(15),11,13-triene-3,6,9-triacetic acide (PCTA), 1,4,7-triazacyclononane N,N',N44-triacétique acide (NOTA), AAZTA (décrit notamment dans WO 2006/00273 formule III page 120 et US 2006/00118830 page 2 et 89), TETA, TETMA, PDTA, et leurs dérivés benzo, LICAM, MECAM, HOPO (DE 102004062258).

Ch peut également être un dérivé de ces composés dans lesquels un ou plusieurs groupes carboxyliques sont sous forme d'un sel, ester, amide correspondant ; ou un composé correspondant dans lequel un ou plusieurs groupes carboxyliques sont remplacés par un groupe phosphonique et/ou phosphinique. On peut aussi utiliser un chélate choisi parmi : DOTA gadofluorines, DO3A, HPDO3A, TETA, TRITA, HETA, DOTA-NHS, M4DOTA, M4DO3A, PCTA et leurs dérivés, avantageusement choisi parmi. DOTA, DTPA, DO3A, HPDO3A, TRITA, TETA, BOPTA, NOTA, PCTA, DOTMA, AAZTA, HOPO et leurs dérivés.

De manière plus large, le ou les chélates formant l'entité signal pourront répondre à la formule du document WO01/60416 suivante :

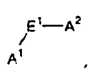
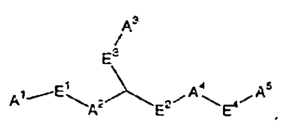
,
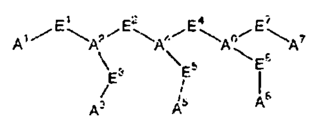
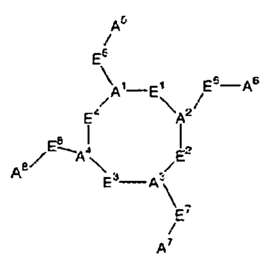
,
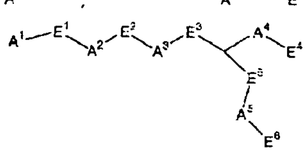
,
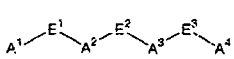
,

On pourra utiliser en particulier les composés DTPA, DOTA, NOTA, DO3A, et dérivés. On citera aussi les chélates rappelés dans WO03/011115 notamment de formules suivantes:

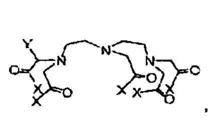
,
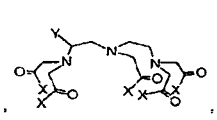
,
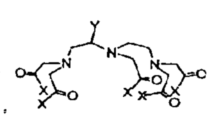
,

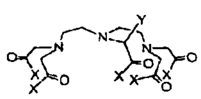 , 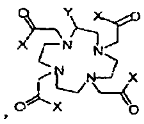 , 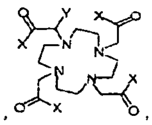 , 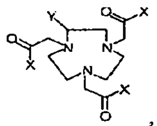 ,

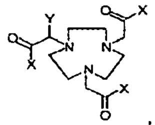 , 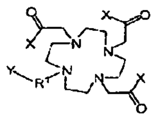 , 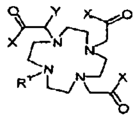 , 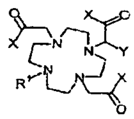 ,

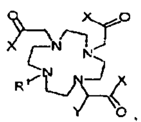 , 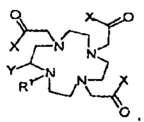 , 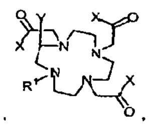 , 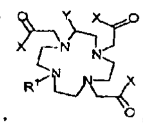 ,

and

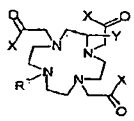 ,

avec X un groupe capable de coordiner un cation métallique, de préférence O-, OH, $NH_2$, $OPO_3$-, NHR avec R une chaîne aliphatique, Y un lien chimique.

On pourra en particulier utiliser les chélates désignés P730 du demandeur décrits dans EP 661 279 (US 5 919 432) de formule

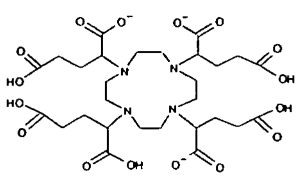

et les chélates à squelette PCTA décrits par le demandeur notamment dans US 6 440 956, que ces chélates ou leurs intermédiaires soient porteurs ou non de chaînes hydrophiles, et en particulier de chaînes aminoalcool courtes ou longues.

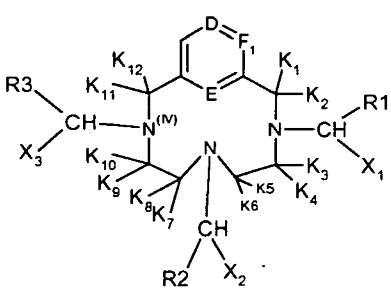

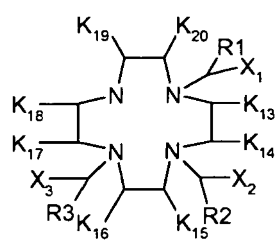

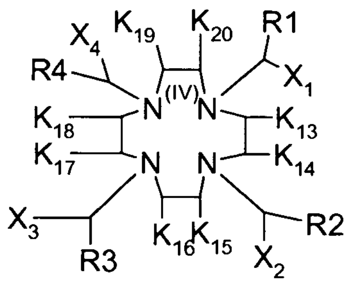 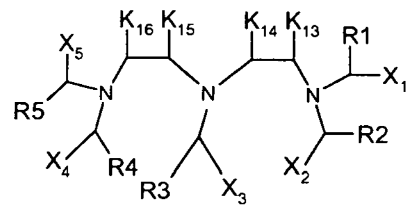

avec X1 à X4 et K1 à K16 des chélates ci-dessus représentant H ou une chaîne en $C_1$-$C_{20}$, et R1, R2, R3, R4, R5 représentant indépendamment -COOH, -P(O)$(OH)_2$ ; et étant choisis de façon à ce que le chélate comprenne au moins une fonction capable d'être couplée à un PEPTIDE VCAM directement ou par l'intermédiaire du Lien.

On citera aussi les chélates du document US2006/0018830 page 9 à 11 de la partie description [0150] à [0158].

Avantageusement dans le cadre de la présente invention, Ch est le DTPA ou le DOTA ou leurs dérivés.

Dans le cas de l'IRM, la relaxivité $r_1$ de ces chélates est typiquement de l'ordre de 4 à 20 $s^{-1}$ $mMol^{-1}$ $Gd^{-1}$ à un champ de 0,5 à 1,5 T. On rappelle que la relaxivité longitudinale $r_1$ d'un produit de contraste paramagnétique donne la mesure de son efficacité magnétique et permet d'apprécier son influence sur le signal enregistré. En imagerie médicale IRM, les produits de contraste modifient le temps de relaxation des protons, et l'augmentation de la relaxivité obtenue permet d'obtenir un signal plus élevé.

On entend dans la formule I par le terme liaison chimique, un groupe de liaison ou Lien L, c'est-à-dire un groupe chimique:

- permettant de relier le Signal et le ou les PEPTIDE VCAM
- n'ayant pas lui-même la fonction d'entité signal qui est assurée par le Signal
- n'ayant pas lui-même la fonction de ciblage qui est assurée par le PEPTIDE VCAM

Le couplage de chélates avec des biovecteurs en particulier peptidiques est décrit dans l'art antérieur, et fait généralement intervenir une liaison chimique (Lien) telle que décrite dans le document WO01/60416. La structure et la nature chimique de la liaison chimique sont définies pour permettre le couplage chimique entre la partie peptidique du PEPTIDE VCAM et le ou les chélates utilisés, et de manière à obtenir une affinité de la partie PEPTIDE VCAM pour sa cible et une spécificité de la reconnaissance appropriée pour l'utilisation.

Un grand nombre de Lien peuvent être utilisés, dans la mesure où ils sont capables d'interagir avec au moins un groupe fonctionnel de biovecteur et au moins un groupe fonctionnel de chélate.

Avantageusement Lien représente :

a) un groupe de formule Q1 -1- Q2,
dans laquelle Q1 et Q2 identiques ou différents représentent O, S, NH, $CO_2$, -NHCO, CONH, NHCONH, NHCSNH, $SO_2$NH- ou $NHSO_2$-, et 1 représente un groupe alkyle (avantageusement en $C_1$-$C_{10}$), alcoxyalkyle (avantageusement en $C_1$-$C_{10}$), alcényle (avantageusement en $C_2$-$C_6$), alcynyle (avantageusement en $C_2$-$C_6$), polyalkoxyalkylène, alkyle interrompu par un ou plusieurs squarate, par un ou plusieurs aryles, avantageusement phényle, ou par un ou plusieurs groupes choisis parmi - NH-, -O-, -CO-, -NH(CO)-, -(CO)NH-, -O(CO)-, ou -(OC)O- ;

b) un groupe $(CH_2)_n$, $(CH_2)_n$-CO-, -$(CH_2)_n$NH-CO- avec n = 1 à 10 , $(CH_2CH_2O)_q(CH_2)_r$-CO- , $(CH_2CH_2O)_q$ $(CH_2)_r$-NH-CO- avec q = 1-10 et r=1-10, $(CH_2)_n$-CONH- , $(CH_2)_n$-CONH-PEG, $(CH_2)_n$-NH-,

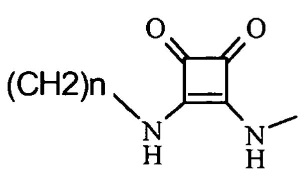 , 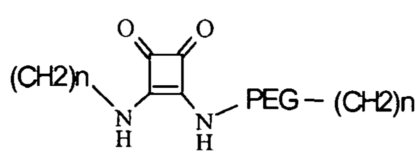 ,

$(CH_2)_n$ - squarate - $(CH_2CH_2O)_q(CH_2)_r$-CO
avec n=1 à 5 et avantageusement n=3, 4, 5,
HOOC-$CH_2$-O-$(CH_2)_2$-O-$(CH_2)_2$-O-$CH_2$-COOH ; HOOC-$(CH_2)_2$-$CO_2$-$(CH_2)_2$-OCO-$(CH_2)_2$-COOH ; HOOC-CH

(OH)-CH(OH)-COOH; HOOC-$(CH_2)_n$-COOH; $NH_2$-$(CH_2)_n$-$NH_2$, avec n=1-20; $NH_2$-$(CH_2)_n$-$CO_2H$ ; $NH_2$-$CH_2$-$(CH_2$-O-$CH_2)_n$-$CO_2H$ avec n=1 à 10.

Notamment :

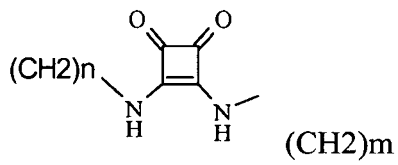

avec n=1 à 4 et m = 0 à 5

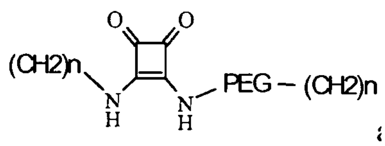

avec n=1 à 4 et m = 0 à 5 (CH2)n - CO avec n= 1 à 5

$(CH_2)_3$ - squarate - $(CH_2CH_2O)_2(CH_2)$-CO

c) des liens décrits dans le brevet US 6 264 914, capables de réagir avec les groupes fonctionnels (du biovecteur et du chélate) amino, hydroxyle, sulfhydryle, carboxyle, carbonyle, carbohydrates, thioéthers, 2-aminoalcohols, 2-aminothiols, guanidinyle, imidazolyle, phénolique ; selon les définitions de ce document.

d) certains liens décrits dans le brevet US 6 537 520 de formule

- $(Cr_6r_7)_g$-$(W)_h$-$(Cr_{6a}r_{7a})_{g'}$-$(Z)_k$-$(W)_{h'}$-$(Cr_8r_9)_{g''}$-$(W)_{h''}$-$(Cr_{8a}r_{9a})_{g'''}$ avec : g+h+g'+k+h'+g"+h"+g"' différent de 0; avec les définitions identiques à celles de ce document colonne 8.

e) certains liens décrits dans le document WO 02/085908 (avec les définitions identiques à celles de ce document), par exemple une chaîne linéaire ou ramifiée de liaison organique choisie parmi :

- CR6"'R7"'-, - (R6"')C=C(R7"')=, -CC-, -C(O)-, -O-, -S-, -$SO_2$-, - N(R3"')-, -(R6"')C=N-, -C(S)-, -P(O0(OR3"')-, -P(O)-(OR3"')O-, avec R"'3 un groupe capable de réagir avec un azote ou un oxygène
- une région cyclique (cycloalkyles divalents, hétérocycles divalents)
- des polyalkylènes, polyalkylènes glycols

f) des liens du document WO03/011115 pages 124-125

g) des liens du document US 2006/0018830 (le Lien du demandeur correspondant au lien désigné N-O-P dans ce document US 2006/0018830)

- liens comprenant au moins un acide aminé non alpha (page 12 à 15, tableau 1 de ce document)
- liens comprenant au moins un acide aminé non alpha porteur d'un groupe cyclique (page 18 à 25, tableau 3 de ce document)
- liens ne comprenant pas d'acide aminé
- autres liens (page 27, 28 de ce document)

Le choix de Lien (structure et taille) pourra se faire notamment de manière à contrôler notamment la charge, la lipophilie, l'hydrophilie du produit de formule (I), pour optimiser le ciblage biologique, la biodistribution. On pourra en particulier utiliser des liens biodégradables in vivo, des liens PEG ou mini-PEG. Le choix du lien est effectué de manière à ne pas altérer de manière gênante l'efficacité du composé de formule (I) selon l'invention, un test permettant de vérifier cette efficacité in vitro et in vivo étant présentés dans la description détaillée.

Selon un autre mode de réalisation, Signal représente un marqueur pour imagerie optique (molécule fluorescente utilisée en imagerie optique). Parmi les marqueurs pour imagerie optique on citera notamment ceux de US2006/0018830 et notamment ceux cités page 11 et 12 paragraphe 1.B, avec des modalités précises d'imagerie décrites colonne 33 ([0259]) au paragraphe 6 (techniques et chromophores et fluorophores décrits en détail).

Selon un autre mode de réalisation, Signal représente des quantum dots (fluorophores inorganiques comprenant des nanocristaux).

Selon un autre mode de réalisation, Signal représente une nanoparticule superparamagnétique revêtue d'une couche organique, communément désignée SPIO ou USPIO (« ultra small particles of iron oxide »). Avantageusement, la nanoparticule comporte un noyau d'oxyde ou d'hydroxyde de fer, notamment de magnétite ($Fe_3O_4$), maghémite ($\gamma$-$Fe_2O_3$). On utilisera avantageusement une nanoparticule recouverte d'un revêtement bisphosphonate, avantageusement gem-bisphosphonate, décrite dans WO2004058275, la particule et la méthode de couplage entre le peptide et la nanoparticule étant détaillées dans les exemples de la présente demande. Les nanoparticules magnétiques utilisées sont des nanoparticules acides à base d'un composé du fer, et recouvertes par une couche comprenant un ou plusieurs composés *gem*-bisphosphonates identiques ou différents, la couche de recouvrement de la nanoparticule ayant la formule (C) suivante :

$$T\text{-}L2\text{-}CH(PO_3H_2)_2 \qquad (C)$$

dans laquelle :

- Le lien L2 représente un groupement organique reliant la fonction T à la fonction *gem*-bisphosphonate $-CH(PO_3H_2)_2$ ;
- T représente une fonction chimique couplé au PEPTIDE VCAM ou au Lien de la présente demande. Dans un mode de réalisation particulier T-L2 représente le Lien du composé de formule (I).

La composition se présente sous forme d'une solution aqueuse stable de nanoparticules. Dans ces compositions, le taux de complexation du composé (C) sur les particules est supérieur à 50%, avantageusement à 70%, et de préférence supérieur à 80, 90, 95%. On préfère particulièrement que les particules magnétiques acides (p) soient complexées sur au moins 90% de leurs sites protonés par des composés de formule (C). Selon une variante, une partie des fonctions T de la couche est couplée à un PEPTIDE VCAM, et une partie des fonctions T est couplée à un composé hydrophile notamment un composé porteur de groupes hydroxyle et notamment un composé hydrophile aminoalcool désigné AAG1AA28 décrit dans WO2004058275 (exemple 8) ou un groupe PEG.

Les particules magnétiques (p) possèdent un diamètre hydrodynamique compris entre 5 et 300 nm, de préférence entre 5 et 60 nm, encore de préférence entre 5 et 30 nm.

Le lien L2 permet de relier et/ou d'espacer la fonction *gem*-bisphosphonate et l'entité réactive T capable d'assurer le greffage covalent du PEPTIDE VCAM (le biovecteur) sur la nanoparticule, par l'intermédiaire ou non du Lien..

A titre préféré, le lien L2 représente un groupement divalent.

De préférence, le lien L2 est choisi parmi :

- un groupe aliphatique ; alicyclique ; alicyclique aliphatique ; aromatique ; aromatique aliphatique, lesdits groupes aliphatiques, alicycliques et aromatiques pouvant être éventuellement substitués par un groupe méthyle, hydroxy, méthoxy, acétoxy, amido, ou un atome d'halogène, avantageusement un atome de chlore, d'iode ou de brome ;
- un groupement $-I_1\text{-}NHCO\text{-}I_2$ où $I_1$ et $I_2$, identiques ou différents, représentent un groupe aliphatique ; alicyclique ; aromatique ; alicyclique aliphatique ou aromatique aliphatique, lesdits groupes pouvant être éventuellement substitués par un groupe méthyle, hydroxy, méthoxy, acétoxy, amido ou un atome de chlore, d'iode ou de brome.

Selon des réalisations préférées, L2 représente un groupe aliphatique substitué ou non, et plus préférentiellement un groupe $-(CH_2)_p-$, où p est un entier de 1 à 5, ou préférentiellement un groupe $-(CH_2)_n\text{-}NHCO\text{-}(CH_2)_m-$ où n et m représentent un nombre entier de 0 à 5.

A titre de groupes T préférés, on peut citer notamment les groupes COOH, $-NH_2$, -NCS, $-NH\text{-}NH_2$, -CHO, alkylpyrocarbonyle (-CO-O-CO-alk), acylazidyle ($-CON_3$), iminocarbonate ($-O\text{-}C(NH)\text{-}NH_2$), vinylsulfuryle ($-S\text{-}CH{=}CH_2$), pyridyldisulfuryle, (-S-S-Py), haloacétyle, maléimidyle, dichlorotriazinyle, halogène, les groupes -COOH et $-NH_2$ étant particulièrement préférés.

De préférence, T représente un groupe -COOH ou $-NH_2$ et L2 un groupe aliphatique substitué ou non, avantageusement un groupe $-(CH_2)_p$ -, où p est un entier de 1 à 5.

On préfère tout particulièrement la couche de formule (C1) suivante

$$HOOC\text{-}(CH_2)_2\text{-}CH(PO_3H_2)_2$$

Plusieurs composés de type nanoparticules d'oxyde de fer porteurs de PEPTIDE VCAM sont décrits dans la description détaillée (notamment PEG - USPIO).

Selon un autre mode de réalisation, Signal représente une nanoparticule lipidique comprenant au moins un chélate. Les nanoparticules lipidiques peuvent se trouver sous la forme d'émulsion nanoparticulaire, contenant ou non des perfluorocarbones, telle que celles décrites dans les documents WO 03/062198, US 5 958 371, US 5 080 885, US

6 403 056.

Les nanoparticules lipidiques peuvent être mises en suspension dans un milieu aqueux ou hydrophile. Ces nanoparticules ont un diamètre de l'ordre de 10 nm à 500 nm, notamment 20 à 250 nm. Les nanoparticules en émulsion peuvent comprendre ou être couplées avec un grand nombre de chélates, par exemple 10 000 à 100 000 chélates par nanoparticule. Les émulsions comprennent suffisamment de composés de formule (I) et donc de Peptide pour permettre la reconnaissance de la zone d'apoptose. On citera comme nanoparticules possibles des liposomes, unilamellaires ou multilamellaires, des micelles, des microgels, des gouttelettes d'huiles, des lipoprotéines, tels que HDL, LDL, IDL, VLDL, chylomicrons, des nanoparticules de fluorocarbone, des nanobulles, ou analogues dont la surface est lipophile.

Avantageusement le chélate est lipophile et attaché à la membrane de la nanoparticule.

De façon avantageuse le Lien du composé de formule (I) est suffisamment lipophile pour coupler le Peptide à la membrane de la nanoparticule lipidique, le PEPTIDE VCAM étant suffisamment exprimé à la partie externe de la nanoparticule pour la reconnaissance spécifique de la cible apoptotique. Le Lien est par exemple un groupe lipophile tel qu'une chaîne alkylène en $C_{10}$-$C_{20}$, cette chaîne s'insérant dans la couche lipidique de la nanoparticule et permettant ainsi d'attacher le Peptide à la nanoparticule.

De nombreux chélates rendus lipophiles pour être associés à une membrane lipidique sont décrits en détail notamment dans les documents US 6 045 821, WO90/04943, WO 2006/100305. Selon des réalisations le chélate porte une longue chaîne lipophile (phospholipide par exemple) qui vient s'insérer dans la membrane de la nanoparticule lipidique (liposome, micelle, nanoémulsion). De même le PEPTIDE VCAM porte avantageusement une chaîne lipophile (le Lien, par exemple une chaîne alkyle en $C_{10}$ à $C_{20}$) qui vient s'insérer dans la membrane de la nanoparticule lipidique.

Selon des réalisations avantageuses, la nanoparticule lipidique inclut des perfluorocarbones tels que décrits dans US 5,958,371, l'émulsion liquide contenant des nanoparticules comportant un perfluorocarbone à point d'ébullition assez élevé (par exemple entre 50 et 90 °C) entouré d'un revêtement composé d'un lipide et/ou d'un surfactant. Le surfactant est capable de se coupler directement à un biovecteur de ciblage ou d'inclure un composé intermédiaire lié de manière covalente au biovecteur, le cas échéant à l'aide d'un agent de liaison chimique.

Différentes émulsions de perfluorocarbone sont rappelées dans le document US 6 676 963 (perfluorodecalin, perfluorooctane, perfluorodichlorooctane, bromure de perfluoro-n-octyl, perfluoroheptane, perfluorodécane, perfluorocyclohexane, perfluoromorpholine, perfluorotripropylamine, perfluorotributylamine, perfluorodiméthylcyclohexane, perfluorotriméthylcyclohexane, éther de perfluorodicyclohexyl, perfluoro-n-butyltétrahydrofuran).

On utilise habituellement comme phospholipides formant la membrane des nanoparticules les composés suivants : phosphatidylcholine dioléoylphosphatidylcholine, dimyristoylphosphatidylcholine, dipalmitoylphosphatidylcholine, distéaroylphosphatidylcholine, phosphatidyléthanolamine.

De telles compositions sont décrites par exemple dans US 5,989,520 et US 5,958,371, comme rappelé dans le document US 20040248856 qui cite notamment des composés perfluorocarbone : perfluorodécalin, perfluorooctane, perfluorodichlorooctane, bromure de perfluoro-n-octyl, perfluoroheptane et analogues.

Selon des réalisations avantageuses, on utilisera pour préparer des agents de contraste selon l'invention des méthodes et des compositions lipidiques appropriées rappelées dans US 6 010 682, notamment pour ce qui concerne la description détaillée de la composition lipidique, et de la préparation de liposomes, de micelles, d'émulsions.

On rappelle que les émulsions sont des mélanges lipidiques hétérogènes obtenues de manière appropriée par agitation mécanique et/ou addition d'agents émulsifiants. Par exemple, on mélange mécaniquement les chélates rendus lipophiles à des solvants organiques tels que le chloroforme. Après évaporation du solvant, les lipides sont remis en suspension en milieu aqueux tel que PBS pour obtenir une émulsion qui subit ensuite typiquement une sonication et une microfluidisation. Les émulsions obtenues peuvent être lyophilisées avec le cas échéant utilisation d'agents anti agglutination.

Pour formuler l'émulsion d'agent de contraste paramagnétique souhaité, on utilise typiquement 1 à 75% en poids de composé chélate lipophile par rapport aux ingrédients totaux de l'émulsion. La composition formant l'agent de contraste est administrée de préférence en intravasculaire, selon le patient examiné, par exemple à raison de 0,1 mg à 1 g de composé chélate lipophile et de 1 à 50 micromoles d'ion de métal paramagnétique par kg de patient.

Les compositions lipidiques obtenues sont le cas échéant formulées à l'aide d'additifs rappelés dans US 6 010 682, notamment pour une administration par injection intraveineuse. On citera notamment le dextrose, le chlorure de sodium, des antimicrobiens.

Avantageusement grâce aux compositions selon l'invention on peut obtenir une augmentation de la relaxivité par ion. On obtient typiquement les caractéristiques suivantes, pouvant varier selon les compositions précises des émulsions et leur procédé de préparation :

- index de polydispersité : 0,2 à 0,3
- $[Gd^{3+}]$ = 2 à 10 mM de préférence 3 à 7 mM
- concentration en particules : 50 à 100 nM
- r1 ($mM^{-1}s^{-1}Gd^{-1}$) : 5 à 40, de préférence 10 à 40

- r2 ($mM^{-1}s^{-1}Gd^{-1}$) : 20 à 40
- r1 ($mM^{-1}s^{-1}$ particule-1) : $10^6$ à $4x10^6$
- nombre de biovecteurs : 50 à 1000, notamment 100 à 300

L'invention concerne aussi ces composés de formule (I) dans laquelle le Peptide contient une séquence peptidique modifiée, mais sans altérer l'affinité et la spécificité pour la cible et l'efficacité du composé in vivo. Le PEPTIDE VCAM est par exemple modifié en utilisant des méthodologies appropriées décrites dans l'art antérieur par exemple dans US2005100963 (colonne 20-21 paragraphes [529] à [541] dans le cas de peptides ciblant des récepteurs KDR), afin de sélectionner des composés de formule (I) efficaces :

1) substitution d'acides aminés sans altérer leur fonction, selon la méthode rappelée dans ce document pour des acides aminés hydrophobe, aromatique, acide, contenant des hydroxyles, contenant des chaînes latérales amide. Pour la lysine on utilisera aussi d'autres acides aminés dibasiques (arginine, histidine, ornithine) ou des dérivés de la lysine ou de ces autres acides aminés, notamment les dérivés alkyles, alcényle, aryle ; tels que des dérivés N-epsilon-isopropyle-lysine, On pourra en particulier tester les dérivés cités au paragraphe [533] de US2005100963.
2) substitution de liaisons amides présentes sur le squelette du polypeptide, notamment pour limiter la dégradation du peptide et/ou ajuster la flexibilité du peptide (par exemple insertion de alpha-N-méthylamide, ou d'une thioamide, remplacement d'un aminoacide par un aza aminoacide)
3) introduction d'acides aminés de la série D, tels que la D-alanine afin de jouer sur l'accessibilité du peptide pour sa cible du fait d'un effet de la modification stérique sur l'orientation des chaînes latérales.
4) modifications chimiques pour ajuster la solubilité et la pharmacocinétique du composé de formule (I), par exemple en ajoutant un groupe hydrophile, ou basique, ou groupe non polaire alkyle ou aromatique), à l'aide d'une liaison à la partie C ou N terminale du peptide, ou au niveau d'une chaîne latérale d'acides aminés du peptide, notamment au niveau de la lysine qui présente une fonction amine libre (ou d'un dérivé tel qu'un 2-,3-diaminopropionique acide).
5) glycosilation
6) autres modifications :

- formation de sels : N-methylglucamine (méglumine), acétate, oxalates, ascorbates, etc
- manipulation de la séquence peptidique (par exemple rétropeptide ou cyclisation).

Les tests biologiques décrits en détail dans la demande permettent de sélectionner des peptides efficaces équivalents ou améliorés en terme d'activité de ciblage de VCAM en comparaison avec les peptides exemplifiés en détail dans la présente demande.

L'invention concerne selon un autre aspect les produits de contraste pour l'IRM comprenant un composé de formule (I) tel que décrit précédemment dont l'ion de métal paramagnétique M est de nombre atomique 21-29, 42-44 ou 58,70, de préférence de gadolinium. Les métaux paramagnétiques M incluent les lanthanides de nombre atomique 58-70 et les métaux de transition de nombre atomique 21-29, 42 or 44, par exemple scandium, titane, vanadium, chrome, manganèse, fer, cobalt, nickel, cuivre, molybdène, ruthénium, cérium, praseodymium, neodymium, prométhium, samarium, europium, gadolinium, terbium, dysprosium, holmium, erbium, thulium, et ytterbium. On préfère particulièrement les éléments Gd(III), Mn(II), europium, dysprosium, avantageusement M est choisi parmi Gd, Mn, Fe, Dy et Tm.

Avantageusement on utilisera les composés Signal -Lien -Peptide pour une imagerie Cest (transfert de saturation) en les intégrant à des composés de type particules lipidiques telles que des liposomes (comme décrit en détail dans WO2006/032705). On rappelle que l'imagerie CEST avec ou sans particules lipidiques et avec ou sans biovecteur peut s'effectuer avec des chélates de valeur q=1 ou de valeur q=2).

L'invention concerne selon un autre aspect les produits de contraste pour l'imagerie aux rayons X ou à la CT, comprenant un composé (I) tel que décrit précédemment dont l'ion de métal lourd M est de nombre atomique 21-31, 39-50 , 56-80, 82, 83 ou 90.

L'invention concerne selon un autre aspect des produits radiopharmaceutiques, comprenant un composé de formule (I) tel que décrit précédemment dont le chélate est couplé avec un radionucléide ou un radiohalogène connu de l'homme du métier, typiquement le gadolinium, le technétium, le chrome, le gallium, l'indium, l'ytterbium, le rhénium, le lanthanium, l'yttrium, le dysprosium, le cuivre ou analogue. Les radionucléides incluent les formes radioactives des éléments Sm, Ho, Y, Pm, Gd, La, Lu, Yb, Sc, Pr, Tc, Re, Ru, Rh, Pd, Pt, Cu, Au, Ga, In, Sn, Cr, Pb, notamment $^{99}Tc$, $^{117}Sn$, $^{111}In$, $^{97}Ru$, $^{67}Ga$, $^{68}Ga$, $^{89}Zr$, $^{177}Lu$, $^{47}Sc$ $^{105}Rh$; $^{188}Re$, $^{60}Cu$, $^{62}Cu$, $^{64}Cu$, $^{67}Cu$, $^{90}Y$, $^{159}Gd$, $^{149}Pr$, $^{166}Ho$; avantageusement $^{68}Ga$. La préparation de composés utilisables comme radiopharmaceutiques notamment en utilisant le technétium $^{99}Tc$ est rappelée dans US 2006/0018830 page 32 paragraphe 4, ces techniques étant décrites dans ce document pour des composés comprenant un chélate couplé à un peptide de ciblage de la gastrine.

Pour des indications en radiothérapie, le couplage de macrocycles de type DOTA, la sélection de nucléides appropriés, la préparation des composés radiothérapeutiques est rappelée dans US 2006/0018830 colonne 35 et 36

(incorporés par référence).

L'invention concerne selon un autre aspect une méthode de diagnostique et une méthode de traitement radiopharmaceutique utilisant un composé de formule (I) tel que décrit précédemment.

La présente invention concerne en outre une composition comprenant au moins un composé de formule générale (I) tel que décrit précédemment et un excipient pharmaceutiquement acceptable, avantageusement destinée à une administration par voie parentérale. Elle concerne de plus un procédé de préparation d'une telle composition comprenant l'addition d'un composé de formule générale (I) tel que défini précédemment à un milieu injectable comprenant l'excipient pharmaceutiquement acceptable.

L'invention concerne l'utilisation d'une composition selon la présente invention pour le diagnostique d'une pathologie associée à VCAM. Les compositions diagnostiques et radiopharmaceutiques selon l'invention peuvent être utilisées comme décrit dans les demandes US 2002/0090342, US 2002/0098149, WO 02/055111 pour des indications anticancéreuses. L'invention concerne de plus les composés de formule générale (I) tels que définis précédemment pour leur utilisation en tant qu'agent de diagnostique de maladies associées à VCAM, avantageusement choisies parmi les maladies cardiovasculaires, les risques d'accident ischémiques, la colite ulcérative chronique, la maladie de Crohn et/ou le cancer, de façon avantageuse parmi les maladies cardiovasculaires, les risques d'accident ischémiques.

De façon avantageuse, la maladie cardiovasculaire est choisie parmi : une maladie associée à des plaques d'athérome, avantageusement des plaques vulnérables, et/ou une maladie coronarienne.

Avantageusement, le risque d'accident ischémique est choisi parmi : l'infarctus du myocarde, un accident vasculaire cérébral, une embolie rénale, une ischémie aiguë d'un membre, et une rupture d'anévrysme aortique.

L'invention concerne aussi l'utilisation des composés décrits précédemment pour la préparation d'une composition diagnostique ou radiopharmacautique destinée au diagnostique et/ou au traitement de maladies associées à l'expression de VCAM, de façon avantageuse choisi parmi les maladies cardiovasculaires et/ou les risques d'accident ischémiques.

Le cas échéant les composés de formule (I) et les peptides VCAM du demandeur seront utilisés en tant qu'agent de diagnostique, ou en tant qu'agent de traitement thérapeutique au niveau de zones d'expression de VCAM, ou en tant qu'agent de diagnostique et de traitement thérapeutique, ou de suivi diagnostique de l'efficacité thérapeutique. Le cas échéant le composé sera co-administré simultanément ou de manière différée avec d'autres agents diagnostiques et/ou thérapeutiques ciblant des zones d'expression de VCAM. L'invention concerne aussi une méthode comprenant la synthèse d'un composé comprenant un métal paramagnétique selon l'invention, capable de cibler une zone pathologique, son administration à un patient, l'imagerie par IRM. L'invention concerne aussi une méthode de diagnostique comprenant la synthèse d'un composé radiopharmaceutique selon l'invention, capable de cibler une zone pathologique, son administration à un patient, l'imagerie par scintigraphie gamma SPECT ou planaire, ou tomographie par émission de positrons.

Pour un diagnostique en IRM, l'administration intraveineuse par injection habituellement en solution saline, se fait typiquement à une dose d'ion métallique de 0,001 à 1,5 mmole/kg de poids corporel, par exemple de 1 à 500 $\mu$mol Gd/kg. Pour un diagnostique radiopharmaceutique, l'administration intraveineuse par injection habituellement en solution saline, se fait typiquement à une dose de 1 à 100 mCi pour 70 kg de poids corporel, de préférence de 5 à 50 mCi, avec une imagerie de diagnostic par exemple 30 à 180 minutes après l'injection pour le $^{99}$Tc.

Pour une utilisation comme agents de contraste aux rayons X , la concentration en atome lourd est typiquement de 0,1 M à 5 M, avec des concentrations par administration intraveineuse de l'ordre de 0,5 à 1,5 mmol/kg.

L'invention concerne selon un autre aspect l'utilisation d'un composé (I) tel que décrit précédemment pour la préparation d'une composition destinée à l'imagerie optique.

Des exemples d'administration de compositions pour l'imagerie médicale sont décrits dans l'art antérieur, par exemple dans le document WO 0226776 et US 2006/0018830 colonne 36 ([0282]) paragraphe 8 (dosages et additives).

Des véhicules pharmaceutiquement physiologiquement acceptables permettant de former des compositions diagnostiques (produits de contraste) comprenant les composés décrits précédemment sont connus de l'art antérieur. On utilisera par exemple des sels (sodium, calcium, méglumine), des agents de contrôle du pH (acide acétique, citrique, fumarique, des antioxydants.

L'invention concerne aussi un procédé de préparation de composés comprenant le couplage d'un PEPTIDE VCAM avec au moins un chélate. Plusieurs méthodes générales de préparation de composés de formule (I) décrites dans le US2006/0018830 (Bracco) sont applicables en utilisant un Peptide à la place des peptides de ces documents. Ces méthodes choisies en fonction notamment du chélate choisi sont rappelées dans 2006/0018830 colonne 37 ([0288] à ([0291]) ("general preparation of compounds », et « alternative preparation of the compounds via segment coupling), par exemple les méthodes SPPS et FMOC (carbamate 9-fluorenylmethyl).

L'invention concerne aussi une méthode de diagnostique ou de traitement radiopharmaceutique comprenant l'administration d'un composé (I), la réalisation d'un examen d'imagerie à l'aide d'un équipement approprié, et l'analyse des résultats.

L'invention couvre sauf indication contraire, toutes les formes chirales, diastéréoisomères, racémiques, notamment cis-trans, L-D des composés décrits.

Le demandeur a aussi étudié les possibilités d'association d'un PEPTIDE VCAM couplé à plusieurs chélates dans le composé de formule (I). Le demandeur a par ailleurs étudié des composés de formule (I) présentant un assemblage entre un ou plusieurs Peptides du composé de formule (I) ciblant VCAM, et un ou plusieurs chélates ; et de manière à ce que l'accès à la cible ne soit pas gêné malgré la présence du ou des chélates. Par exemple, le chélate est espacé du ou des PEPTIDES P par le Lien d'une taille suffisante et d'une structure chimique telles que la reconnaissance du ou des peptides par leur cible n'est pas altérée.

Parmi les associations biovecteurs (peptides ou éventuels d'autres biovecteurs) /chélates, on peut citer notamment :

- un peptide biovecteur central relié à plusieurs chélates identiques ou différents
- un chélate central relié à plusieurs peptides identiques ou différents
- un premier ensemble [peptide porteur de chélate(s)] couplé par l'intermédiaire d'un lien hydrophobe ou hydrophile à un second ensemble [peptide porteur de chélates(s)], s'écrivant par exemple :

    Ch1-peptide1-Lien-peptide2-Ch2 avec Ch représentant des chélates identiques ou différents et peptide1 et peptide2 représentant des Peptides identiques ou différents

- un ensemble peptide1-(Lien porteur de Ch)-peptide2-Ch On utilisera par exemple la méthode de construction de composés multimériques décrite dans US2005/0100963 (WO2006/031885 page 66 ligne 25 à page 69 ligne 30) dans le cas de peptides ciblant des récepteurs KDR (par exemple à l'aide de la méthode 13 des exemples : « preparation of homodimers and heterodimers »), mais en utilisant le PEPTIDE VCAM (on remplacera par exemple les peptides des composés des figures 44 à 47 du US2005100963 par des PEPTIDES VCAM). Le composé peut ainsi avantageusement comprendre un peptide couplé à plusieurs chélates, ou un chélate couplé à plusieurs peptides identiques ou différents. L'invention concerne aussi des composés mixtes comprenant en plus du PEPTIDE VCAM au moins un autre biovecteur ciblant VCAM, le biovecteur étant soit un autre peptide soit un autre biovecteur mais non peptidique.

On pourra le cas échéant coupler la partie peptide ou la partie chélate à des groupements chimiques permettant de favoriser la biodistribution, la durée de vie du produit dans le sang.

La spécificité du produit fait référence à son affinité spécifique pour au moins un marqueur de VCAM ou de tout trouble pathologique associé, la spécificité de la liaison exprimée typiquement par des constantes Kd et Ka, la valeur Kd pour les marqueurs cibles étant inférieures à 10 μM, de préférence inférieure à 1 μM.

Parmi les sels pharmaceutiquement acceptables, on citera notamment des sels de cations de bases inorganiques (potassium, sodium, calcium, magnésium...), des cations de bases organiques (éthanolamine, diéthanolamine, morpholine, glucamine, N-méthylglucamine, N, N-diméthylglucamine...), des anions d'acides inorganiques (notamment des chlorures, bromures, iodures, sulfates...), des anions d'acides organiques (acétate, succinate, citrate, fumarate, maléate, oxalate, trifluoroacétate...), des ions d'acides aminés (taurine, glycine, lysine, arginine, ornithine, acide aspartique, acide glutamique...).

L'invention concerne aussi l'utilisation d'un PEPTIDE VCAM pour un diagnostique in vitro (dosage du VCAM-1 soluble dans le plasma sanguin) et pour la thérapie anti-inflammatoire (une multimérisation du peptide peut augmenter son efficacité par l'effet de polyvalence).

Définitions :

On reprend ici les définitions de pathologies dont le diagnostique est l'objet de la présente invention, exposées dans le document WO200603788.

Par « peptide ciblant VCAM » ou "PEPTIDE VCAM" on désigne une molécule capable de se lier de manière sélective à VCAM (plus spécialement VCAM-1). Par "maladie cardiovasculaire" on inclut notamment les états marquant le développement d'une plaque et les complications découlant de la formation d'une plaque d'athérome (sténose, ischémie) et/ou de son évolution vers un accident ischémique aigu (thrombose, embolie, infarctus, rupture artérielle). Les maladies cardiovasculaires désignent par exemple l'athérosclérose, une plaque d'athérome, en particulier la plaque vulnérable, la maladie coronarienne, l'angor, la thrombose, l'accident vasculaire cérébral, l'infarctus du myocarde, la sténose vasculaire, l'infarctus.

La "maladie coronarienne" est la manifestation la plus courante de la maladie cardiovasculaire. Il s'agit d'une maladie progressive, due à une mauvaise irrigation du muscle cardiaque, consécutive au rétrécissement (sténose) ou à la calcification (sclérose) d'une ou des artères coronaires. Le symptôme principal de la maladie coronarienne se manifeste sous la forme de douleurs qui constituent un angor (stable ou instable), aussi appelé angine de poitrine.

L'obstruction complète d'une ou des artères coronaires conduit à l'infarctus.

L'"infarctus" désigne un foyer circonscrit de nécrose dû à une obstruction artérielle. Plus spécifiquement, l'infarctus du myocarde est une nécrose du myocarde qui résulte généralement d'une thrombose coronaire aiguë, secondaire à une rupture de plaque (généralement une plaque instable ou plaque vulnérable) entraînant l'agrégation plaquettaire puis l'occlusion coronaire.

La "thrombose" correspond à la coagulation du sang dans les cavités vasculaires (artères, veines, capillaires ou cavités cardiaques) conduisant à la formation d'un thrombus.

L'"embolie" est la migration intravasculaire d'un corps étranger, le plus souvent constitué par un caillot sanguin (thrombus), et son arrêt brusque dans un vaisseau dont le calibre est insuffisant pour le laisser passer. Les conséquences locales de l'embolie sont des perturbations circulatoires liées à l'obstruction vasculaire, conduisant le plus souvent à un infarctus.

L'"ischémie" désigne la diminution de l'apport sanguin artériel dans un territoire de l'organisme. Ses principales causes locales sont la thrombose et l'embolie. L'expression "plaque vulnérable" désigne une plaque d'athérome présentant une chape fibreuse fine (environ 65 à 150 [mu]m d'épaisseur) et un coeur lipidique important. Ces plaques instables, qui présentent une tendance à la rupture, se rencontrent au niveau des artères coronaires et au niveau de l'aorte et de ses branches. La rupture des plaques coronariennes vulnérable provoque des "syndromes coronariens aigus" : en cas de thrombose occlusive complète, il s'agit de l'infarctus du myocarde ; lorsque la thrombose de l'artère reste incomplète il s'agit de l'angor instable. Au niveau de la carotide, les plaques vulnérables sont plus sténotiques et moins inflammatoires. Elles expriment également VCAM-1. Dans la présente demande on utilise le tableau de correspondance suivant.

| | | |
|---|---|---|
| Alanine | A | Ala |
| Arginine | R | Arg |
| Asparagine | N | Asn |
| Aspartate | D | Asp |
| Cystéine | C | Cys |
| Glutamate | E | Glu |
| Glutamine | Q | Gln |
| Glycine | G | Gly |
| Histidine | H | His |
| Isoleucine | I | Ile |
| Leucine | L | Leu |
| Lysine | K | Lys |
| Méthionine | M | Met |
| Phénylalanine | F | Phe |
| Proline | P | Pro |
| Sérine | S | Ser |
| Thréonine | T | Thr |
| Tryptophane | W | Trp |
| Tyrosine | Y | Tyr |
| Valine | V | Val |

Comme illustré plus loin, le demandeur a démontré notamment l'efficacité de produits pour IRM comprenant un chélate lié au PEPTIDE VCAM notamment NNSKSHT.

Ainsi quand bien même un peptide serait connu de l'art antérieur, l'identification de son utilité dans un mécanisme de ciblage de VCAM, en particulier de VCAM-1, parmi le nombre extrêmement important de cibles biologiques possibles, est loin d'être évidente. De plus il n'est nullement évident que cette cible biologique identifiée, des composés couplés (PEPTIDE VCAM - entité signal) permettent de résoudre les problèmes techniques résolus par le demandeur,

notamment :

- la conservation de l'affinité in vivo pour le site reconnaissance biologique, malgré l'encombrement stérique et la modification possible de conformation in vivo du fait du couplage à une entité signal
- la possibilité de couplage chimique avec les entités signal ; le couplage avec les dérivés PCTA en particulier a impliqué la mise au point d'une synthèse chimique dédiée
- la stabilité physico-chimique in vivo qui constitue une limitation majeure pour de nombreux peptides
- la capacité d'être reconnu en imagerie in vivo, et cela en particulier en IRM, technique dont le niveau de sensibilité est près de 1000 fois inférieur à l'imagerie PET.

Au global, il n'était donc absolument pas évident pour l'homme du métier :

- d'une part de sélectionner les peptides objet de la présente invention
- d'autre part que les produits intégrant ces peptides soient in vivo effectivement efficaces.

Dans la description détaillée qui suit, on décrit des peptides dont on a montré l'efficacité avec des variantes de réalisation sur différentes entités signal (PARTIE I) et les résultats biologiques (PARTIE II),

La figure 1 illustre le calcul de la valeur Kd* (constante apparente de dissociation) de VCAM1.
La figure 2 représente la mesure de l'affinité du peptide NNSKSHT (SEQ ID No3) (constante d'inhibition-50 ou $IC_{50}$ évaluée).
Les figures 3 et 4 illustrent le rehaussement en IRM d'un produit DOTA-NNSKSHT.
La figure 5 montre des images IRM sur souris ApoE/c obtenues avec un produit DOTA-NNSKSHT.

EXEMPLES PARTIE I : préparation des composés incluant des PEPTIDES VCAM.

Généralités

M : concentration molaire (mole/l).
M/z : masse sur charge déterminée par spectrométrie de masse.
$ES^+$ : électrospray mode positif.
$ES^-$ : électrospray mode négatif.
kDa : unité de masse moléculaire (kiloDalton)
CCM : Chromatographie sur Couche Mince
Z ave : diamètre hydrodynamique mesuré par PCS

Dosage du Fer total:

Le fer est dosé par spectroscopie d'absorption atomique (Spectrophotomètre VARIAN AA10) après minéralisation par HCl concentré et dilution par rapport à une gamme étalon d'ions ferrique (0, 5, 10, 15 et 20 ppm).

Taille des particules :

Diamètre hydrodynamique de la particule greffée (Z ave) :

Déterminé par PCS (appareil Malvern 4700, laser 488 nm à 90°) sur un échantillon dilué à ~ 1 millimolaire avec de l'eau PPI filtrée sur 0.22 μm.
PCS = Photon Correlation Spectroscopy = Technique par Diffusion de Lumière Dynamique - Référence : R. Pecora dans J. of Nano. Res. (2000), 2, p 123-131.

Analyses structurales :

Par spectroscopie de masse (appareil MICROMASS VG Quattro II) avec une source Electrospray.

Mesures de relaxivité :

Les temps de relaxation T1 et T2 ont été déterminés par des procédures standards sur un appareil Minispec 120 (Bruker) à 20 Mhz (0,47T) et 37°C. Le temps de relaxation longitudinal T1 est mesuré en utilisant une séquence d'Inversion Récupération et le temps de relaxation transverse T2 est mesuré par une technique CPMG.

Les vitesses de relaxation R1 (=1/T1) et R2 (=1/T2) ont été calculées pour différentes concentrations en métal total (variant de $0,1.10^{-3}$ à $1.10^{-3}$ mole/L) en solution aqueuse à 37°C. La corrélation entre R1 ou R2 en fonction de la concentration est linéaire, et la pente représente la relaxivité r1 (R1/C) ou r2 (R2/C) exprimées en (1 / seconde) x (1/ mMole/L) soit ($s^{-1}.mM^{-1}$).

Les nanoparticules ont été préparées selon les méthodes décrites dans le brevet WO2004/058 275 (US 2004/253181) exemples 8 et 9 pour la préparation des solutions colloïdales de particules magnétiques et exemples 10 à 12 pour la complexation des particules magnétiques par un revêtement gem-bisphosphonate de l'exemple 1 de WO2004/058 275.

**Exemple 1** : Couplage des peptides sur la particule d'oxyde de fer (produit PEG - USPIO - PEPTIDE VCAM)

Les séquences peptidiques d'intérêt sont présentées dans le tableau suivant :

| N° | Code | peptide | séquence |
|---|---|---|---|
| 1 | COMPOSE A (peptide CNNSKSHTC, Lien L dé type squarate) | 8-Amino-3,6-dioxaoctanoyl-cyclo-[Cys-Asn-Asn-Ser-Lys-Ser-His-Thr-Cys]-OH | CNNSKSHTC (SEQ ID No2) |
| 2 | COMPOSE B (composé A avec séquence mélangée) | 8-amino-3,6-dioxaoctanoyl -His-Ser-cyclo-[Cys-Asn-Lys-Asn-Ser-Cys]-Thr-OH | HSCNKNSCT |

**Couplage du peptide N°1 sur une particule d'oxyde de fer**

Protocoles :

| N° | code | peptide | Masse engagée |
|---|---|---|---|
| 1 | COMPOSE A | 8-Amino-3,6-dioxaoctanoyl-cyclo-[Cys-Asn-Asn-Ser-Lys(tfa)-Ser-His-Thr-Cys]-OH | 20 mg |

*Couplage :*

15 ml de nanoparticules ([Fe]=0,338 M) sont agités à température ambiante, le pH est égal à 7,2. Une solution du peptide protégé (N°1, -Lys(tfa)-, 20mg) dans 1ml d'eau est ajoutée par portion de 100µl avec 2,25mg d'EDCI toutes les 15 minutes. A la fin de l'addition, la solution est maintenue sous agitation 1 nuit. Le pH est ajusté à 7 avec une solution de NaOH 0,1M.

La solution est ensuite filtrée sur 0,22 µm (filtre Durapore Millipore®) et ultrafiltrée sur une membrane de 30 kDa, puis concentrée jusqu'à un volume final de 15 ml.

*Couplage du PEG :*

A 15 ml de la solution précédente est ajouté 2,5 ml d'une solution de 1,060g d'aminoPEG (O-(2-aminoethyl)-O'-methylpolyethyleneglycol 750, Aldrich, RN [80506-64-5]) dans 5 ml d'eau. Le pH de la solution est ajusté à 8 avec HCl 1M, puis 0,325g d'EDCI est ajouté et le mélange est agité 3h. L'ajout de la solution d'aminoPEG (2,5ml) et d'EDCI (0,325g) est renouvelé une fois et le mélange est agité à température ambiante une nuit. Le pH est ramené à 7,5 avec HCl 1M. La solution est filtrée sur 0,22 µm et ultrafiltrée sur membrane de 30 kDa. Le volume final de solution est de 15 ml.

*Déprotection :*

La solution est ajustée à pH 10,1 avec NaOH 1M et agitée à température ambiante pendant 6h. Le pH est ensuite ramené à 7,5 par HCl 1M et la solution est filtrée sur 0,22μ et ultrafiltrée sur membrane de seuil de coupure 12kDa.

*Caractérisation:* PCS : Z ave = 26 nm
Concentration en fer : 137,61 mM
$r_1$ (20MHz, 37°C) : 30,54 $s^{-1}mM^{-1}$
$r_2$ (20MHz, 37°C) : 76. $s^{-1}mM^{-1}$
$r_1$ (60MHz, 37°C) : 14,94 $s^{-1}mM^{-1}$
$r_2$ (60MHz, 37°C) : 85,06 $s^{-1}mM^{-1}$

| Echantillons | Diamètre magne. | Aimantation à saturation (magn.). | Diamètre relax. | Aimantation à saturation (relax.) |
|---|---|---|---|---|
| PEG750-COMPOSE A | 9,94 nm | 63,1 $Am^2/Kg$ | 11,2 nm | 48,9 $Am^2/Kg$ |

Le même protocole a été suivi pour l'obtention des autres produits.

**Couplage du peptide N°2 sur une particule d'oxyde de fer**

| N° | code | peptide | Masse engagée |
|---|---|---|---|
| 2 | COMPOSE B | 8-amino-3,6-dioxaoctanoyl -His-Ser-cyclo-[Cys-Ans-Lys(tfa)-Asn-Ser-Cys]-Thr-OH | 20 mg |

*Caractérisation:*

PCS : Z ave = 29 nm
Concentration en fer: 118,60 mM
$r_1$ (20 MHz, 37°C) : 31,34 $s^{-1}$ $mM^{-1}$
$r_2$ (20 MHz, 37°C) : 79,61 $s^{-1}$ $mM^{-1}$
$r_1$ (60 MHz, 37°C) : 14,30 $s^{-1}$ $mM^{-1}$
$r_2$ (60 MHz, 37°C) : 78,05 $s^{-1}$ $mM^{-1}$

**<u>Exemple 2 : couplage des peptides sur des chélates de gadolinium</u>**

*Schéma général :*

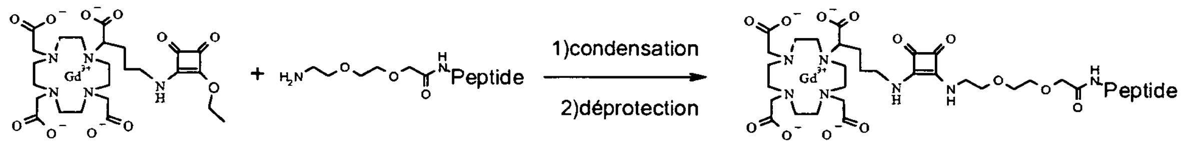


Gd-DOTA-EthylSquarate

*Séquence des peptides couplés :*

| N° | code | peptide | séquence |
|---|---|---|---|
| 1 | COMPOSE A | 8-Amino-3,6-dioxaoctanoyl-cyclo-[Cys-Asn-Asn-Ser-Lys-Ser-His-Thr-Cys]-OH | CNNSKSHTC (SEQ ID No2) |
| 2 | COMPOSE B | 8-amino-3,6-dioxaoctanoyl -His-Ser-cyclo-[Cys-Asn-Lys-Asn-Ser-Cys]-Thr-OH | HSCNKNSCT |

*Condensation, déprotection :*

210 mg de Gd-DOTA-diéthylsquarate (préparé selon le protocole détaillé à l'exemple 3) sont dissous dans 20 ml d'eau, le pH est ajusté à 9 avec une solution saturée de $Na_2CO_3$. 350 mg de peptide protégé N°1 (-Lys(tfa)-) sont ajoutés et le pH est ajusté à 9,2. On laisse réagir 4 jours. La solution est dialysée sur des membranes de seuil de coupure1000 Da pendant 48h puis chromatographiée sur colonne RP-18 (Eluant : MeOH/eau (50/50)).

Les peptides N°2 et 3 sont condensés selon le même mode opératoire.

Les produits obtenus ont pour structure :

| N° | Structure | PM | Spectro.d e masse |
|---|---|---|---|
| 4 | 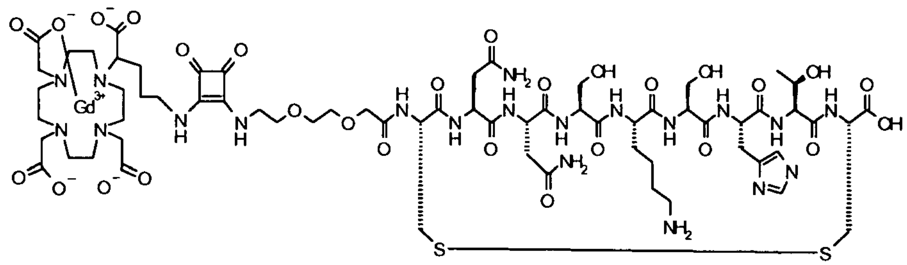 | 1829,0 | conforme |
| 5 | 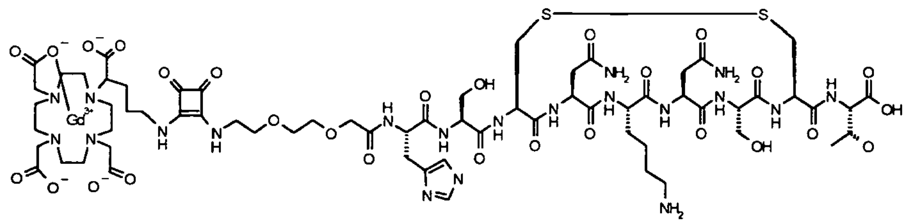 | 1829,0 | conforme |
| 6 | 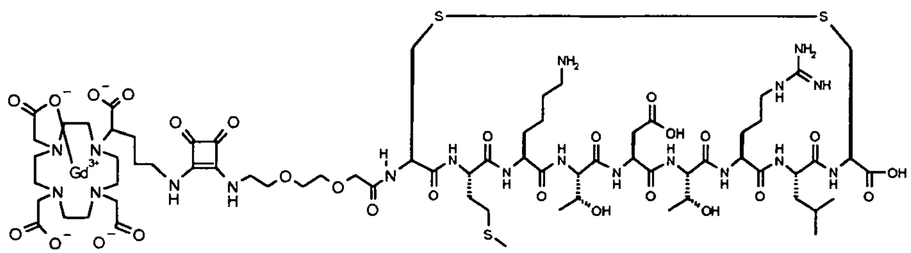 | 1906,23 | conforme |

**Exemple 3 : synthèse d'un chélate bifonctionnel dérivé du DOTA**

Etape 1 : Ester benzylique de l'acide 5-(1,3-Dioxo-1,3-dihydro-isoindol-2-yl)-2-(1,4,7,10 tétraaza-cyclododéc-1-yl)-pentanoique

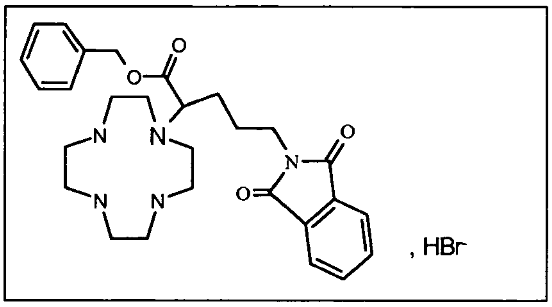

55 g de cyclen base (320 mmol) sont dissous dans 550 mL de $CH_3CN$, auxquels sont ajoutés goutte à goutte 119,8 g de dérivé bromé (2-Bromo-5-(1,3-dioxo-1,3-dihydro-isoindol-2-yl)-pentanoique acide benzyle ester, 288 mmol) dissous dans 550 mL de $CH_3CN$ Le milieu est agité à température ambiante 1 nuit. Le précipité est filtré et lavé abondamment à l'acétonitrile. Obtention de 138 g de produit sous forme d'une poudre blanche (rendement corrigé 81,3 %).
CCM : $CH_2Cl_2$/ MeOH/ $NH_4OH$ à 25% (80/40/3)
Révélation UV et $CuSO_4$
Rf : 0,3

Etape 2 : Ester benzylique de l'acide 5-(1,3-Dioxo-1,3-dihydro-isoindol-2-yl)-2-(4,7,10-tris-éthoxycarbonylméthyl-1,4,7,10tétraaza-cyclododéc-1-yl)-pentanoique

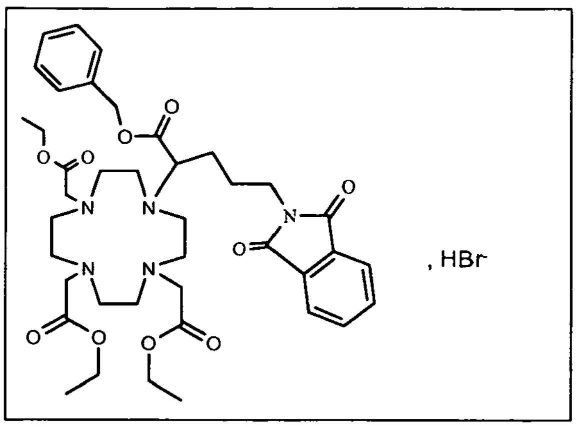

A une solution de 59,1 g d'ethylbromoacétate (Aldrich®, 358 mmol) dans le $CH_3CN$ (1,11) sont additionnées 60 g du composé obtenu à l'étape 1 (102 mmol) et 50,1 g de $Na_2CO_3$ (464 mmol). Le milieu réactionnel est chauffé à 80°C sous couverture d'argon pendant une nuit. Après élimination du précipité, le filtrat est concentré et lavé abondamment au $CH_3CN$. Le produit est cristallisé dans $CH_3CN$ par ajout goutte à goutte d'$Et_2O$. Obtention de 89,8 g de produit sous forme d'un solide blanc (rendement corrigé 100 %).
CCM : $CH_2Cl_2$/ MeOH (9/1)
Révélation UV et $KMnO_4$; Rf : 0,4

Etape 3: Acide 5-Amino-2-(4,7,10-tris-carboxyméthyl-1,4,7,10tétraaza-cyclododéc-1-yl) pentanoique

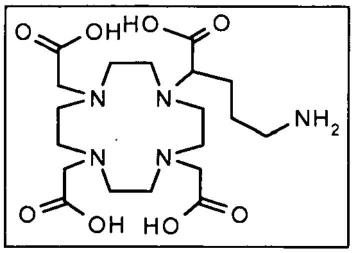

Dans le réacteur de 5 litres, on chauffe au reflux une solution de 54 g de composé obtenu à l'étape 2 (64 mmol) dans l'acide chlorhydrique 37 % (1,8 1), pendant une nuit. Après refroidissement et filtration, le filtrat est concentré et purifié sur silice silanisée (élution à l'eau). Après évaporation sous pression réduite, le produit est lavé à l'éther. Obtention de 45 g de produit sous forme d'un solide blanc. Le produit est désalifié par passage sur résine $OH^-$. On isole 30 g de produit sous forme de cristaux blancs (rendement 100 %).
HPLC : Hypercarb® 5μ, 200X4.6, 250Å ; Solvant A : acide sulfurique 0,037 N
Solvant B : $CH_3CN$ ; Détection UV à 201 nm ; Tr : 18 min.

Etape 4: Complexe de gadolinium de l'Acide 5-Amino-2-(4,7,10-tris-carboxyméthyl-1,4,7,10tétraaza-cyclododéc-1-yl) -pentanoique

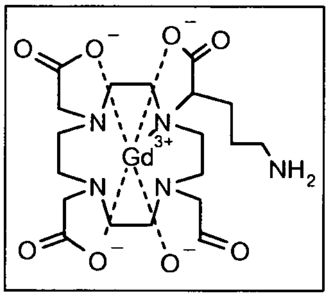

7,2 g du composé obtenu à l'étape 3 (16 mmol) sont dissous dans 70 mL d'eau et le pH est ajusté à 5,5 par ajout d'acide chlorhydrique 6 N. On ajoute 2.9 g de $Gd_2O_3$ (8 mmol), et on chauffe à 80°C le milieu réactionnel. Le pH de la solution augmente régulièrement, et doit être maintenu entre 5,2 et 5,7 par ajout goutte à goutte d'acide chlorhydrique 6 N. Après deux heures, le pH se stabilise à 5,7. Le léger trouble est filtré sur filtre Whatman® et le filtrat est concentré. Obtention de 11,1 g de produit sous forme de paillettes blanches (rendement corrigé 100 %).
HPLC : Hypercarb® 5μ, 200X4,6, 250Å ; Solvant A : acide sulfurique 0,037 N
Solvant B : $CH_3CN$ ; Détection UV à 201 nm ; Tr : 10 min.

Etape 5 : Complexe de gadolinium de l'Acide 5-(2-Ethoxy-3,4-dioxo-cyclobut-1-énylamino)-2-(4,7,10-tris-carboxyméthyl-1,4,7,10tétraaza-cyclododéc-1-yl)-pentanoique

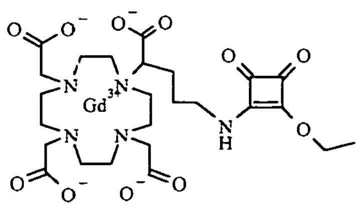

8 g de composé obtenu à l'étape 4 sont séchés par distillation azéotropique au toluène, puis mis en suspension dans 90 ml de DMSO anhydre sous couverture d'argon. 2,8 ml de $Et_3N$ séchée sur tamis (1,7 éq) et 5 g de diéthylsquarate (Aldrich®, 2,5 éq.) sont ensuite ajoutés. Le milieu est agité à température ambiante sous couverture d'argon pendant 1 heure. Le mélange est précipité dans 600 ml d'éther. Le solide obtenu est filtré, puis lavé au dichlorométhane. Après filtration et séchage, 7,5 g d'un solide blanc (rendement de 81,5 %)sont récupérés.
HPLC : Symmetry C18, 5μ, 250X4,6, 100Å
A : eau TFA, pH = 2,7 ; B : $CH_3CN$ ; Détection à 201 et 254 nm ; Tr : 19,8 min.

<u>EXEMPLES PARTIE II : mise en évidence de l'efficacité de PEPTIDES VCAM</u>

Dans toute la PARTIE II qui suit ainsi que dans les figures associées, le peptide cyclique CNNSKSHTC (SEQ ID No2) est écrit sous la forme NNSKSHT (SEQ ID No3) qui montre la séquence.

**<u>II.1 Peptide NNSKSHT(SEO ID No3)</u>**

L'interaction entre le peptide et VCAM-1 est évaluée à l'aide d'un test ELISA. Le peptide NNSKSHT (SEQ ID No3) est immobilisé sur plaque à la concentration 50 μg/mL (3 μg/puits) pendant toute une nuit à 4°C. Les sites non spécifiques sont ensuite saturés dans un tampon enrichi en BSA pendant 2 heures à 4°C. Après plusieurs rinçages, la molécule VCAM-1 (chimère VCAM-1/Fc recombinante humaine) est incubée à des concentrations croissantes ($3{,}9.10^{-9}$ - $4{,}9.10^{-7}$ M) pendant 2 heures à température ambiante. La protéine VCAM-1 liée au peptide est détectée par ajout d'une solution d'anticorps monoclonal de souris anti-VCAM-1 humain diluée au 1/500 dans un tampon TBS. L'incubation se prolonge pendant 1 heure à température ambiante. Après élimination des anticorps non liés, la révélation est effectuée par addition d'un anticorps (secondaire) anti-IgG souris conjugué à la peroxydase dilué au 1/200 dans un tampon phosphate (1 heure à 4°C) et d'une solution de substrat ABTS enrichie en $H_2O_2$ La mesure de la DO à 405 nm permet le calcul de la valeur $K^*_d$ de VCAM-1.
L'affinité du peptide pour VCAM-1 est directement calculée à partir d'un test fonctionnel cellulaire qui consiste à mesurer l'inhibition de l'adhésion cellulaire.
La protéine VCAM-1 est immobilisée sur plaque ELISA à la concentration 20 μg/mL (1,2 μg/puits) pendant une nuit à 4°C. Le peptide NNSKSHT (SEQ ID No3) est alors pré-incubé à des concentrations croissantes pendant 1 heure à température ambiante. Après lavage, des cellules Jurkat, pré-stimulées avec 50 ng/mL de PMA pendant 3 heures, sont incubées ($10^5$ cellules/puits) pendant 1 heure à température ambiante. Après plusieurs rinçages, les cellules liées au VCAM-1 sont fixées dans une solution de glutaraldéhyde 1% (45 minutes à 4°C). Après lavages, une solution de violet de crésyle 1% est incubée pendant 30 minutes. Les puits sont de nouveau rincés puis incubés toute une nuit à température ambiante avec une solution d'éthanol. La coloration bleue des cellules adhérentes permet une mesure de la DO à 630 nm. L'inhibition de l'adhésion cellulaire par le peptide NNSKSHT (SEQ ID No3) permet de calculer une valeur $IC_{50}$.

**<u>II.2. Produit de contraste Gd-DOTA-NNSKSHT</u>**

Le peptide NNSKSHT (SEQ ID No3) protégé par du TFA est couplé au DOTA. La déprotection des lysines est réalisée une fois le couplage effectué.
Avant l'acquisition IRM, les souris sont anesthésiées au pentobarbital. Toutes les expériences sont réalisées sur spectromètre Bruker de 200 MHz (4,7 T) équipé d'un aimant vertical et d'un système de micro-imagerie.
Les produits de contraste sont testés sur deux modèles différents.

**<u>II.2.1 Test sur modèle d'hépatite induite par la concanavaline-A (Con-A) chez la souris</u>**

L'hépatite est induite par injection i.v. de 20 mg/kg de Con-A chez des souris Balb/c d'environ 26 g. La littérature rapporte que VCAM-1 est massivement exprimée dans le foie entre 4 et 8 heures après l'injection de Con-A. Le protocole

d'IRM est le suivant : (1) l'IRM pré-contraste commence 4h30 après l'injection de Con-A; (2) le produit de contraste est injecté à la dose 100 μmol Gd/kg environ 5h après l'injection de Con-A ; (3) plusieurs acquisitions post-contraste sont réalisées pendant 1h30 toutes les 7 minutes. Les images sont acquises à l'aide d'une séquence MSME (multi-slice-multi-echo) dont les paramètres sont les suivants : TR/TE = 307,4/14,7 ms, matrice = 256, FOV = 5 cm, épaisseur de la coupe = 3 mm, 8 coupes axiales (qui couvrent complètement la région abdominale incluant les reins), TA = 5 minutes 14 secondes.

**II.2.2. Test sur modèle d'athérosclérose chez la souris apoE$^{-/-}$**

Des souris femelles C57B16 ApoE$^{-/-}$ âgées environ de 15 mois sont soumises à un régime riche en cholestérol pendant 3 mois avant les études IRM. Pour l'acquisition par IRM les animaux sont anesthésiés. L'agent de contraste Gd-DOTA-NNSKSHT est injecté à la dose de 100 μmol Gd/kg. Toutes les images sont acquises au niveau de l'aorte abdominale, en particulier la région proche du rein qui est connue pour le développement de plaques d'athérome en raison de la présence des branches artérielles.

Les images sont acquises à l'aide de deux séquences :

- MSME (multi-slice-multi-echo) avec les paramètres suivants : TR/TE = 695,8/8,9 ms, NEX = 2, largeur du spectre = 50 kHz, matrice = 256x256, FOV = 2,3x2,3 cm, épaisseur de la coupe = 1 mm, 20 coupes axiales, résolution spatiale = 90x90 μm, TA = 5 minutes 56 secondes.
- Les paramètres de la séquence RARE (rapid acquisition with relaxation enhancement) sont ajustés en utilisant un tube référence rempli d'une solution 1 mM de Gd-DOTA. Les paramètres d'acquisition d'images sont les suivants : TR = 470,9 - 1048,5 ms, TE = 4 ms, facteur RARE = 1 - 4, NEX = 4, matrice = 256 x 256, FOV = 2,3 cm, largeur spectrale = 33,33 kHz, épaisseur de la coupe = 0,8 mm, résolution spatiale = 90 μm.

**II.2.3. Traitement du signal**

Pour les deux modèles, les valeurs SI sont mesurées dans différentes régions d'intérêt (au niveau de la paroi artérielle de l'aorte abdominale ou foie entier) à l'aide du logiciel d'analyse d'image OSIRIS. Les régions sont d'abord dessinées sur les images post-contraste, puis dupliquées sur les images pré-contraste. La valeur SI est mesurée sur toutes les coupes d'images où la paroi artérielle et le foie sont visibles. Finalement, les valeurs SI obtenues pour des coupes sériées d'aorte sur une longueur de 3,2 - 8 mm sont moyennées pour chaque animal. L'augmentation du rapport signal/bruit (ΔSNR %) est calculée selon l'équation suivante :

$$\Delta SNR\ \% = [(SI_{post} / SD_{bruit}) - (SI_{pré} / SD_{bruit})]/[SI_{pré} / SD_{bruitt}]\ X\ 100$$

où $SD_{bruit}$ est la dérivation standard du bruit mesuré sur une région en dehors de l'animal.

*Références*

Wolf D, Hallmann R, Sass G, Sixt M, Küsters S, Fregien B, Trautwein C and Tiegs G (2001) TNF-α-induced expression of adhésion molécules in the liver is under the control of TNFR1-relevance for concanavalin A-induced hepatitis 1, J. Immunol. 166 : 1300 - 1307.

Burtea C, Laurent S, Vander Elst L, Toubeau G and Muller RN (2006) Molecular imaging of angiogenic blood vessels in vulnérable atherosclerotic plaques with a mimetic of RGD peptide grafted to Gd-DTPA, ESMRMB (Annual Meeting of the European Society of Magnetic Résonance in Medicine and Biology), Warsaw, Poland.

**II.3 Résultats**

**II.3.1 Peptide NNSKSHT** (SEQ ID No3)

Le test ELISA (figure 1) basé sur l'immobilisation du peptide NNSKSHT (SEQ ID No3) en présence de concentrations croissantes de VCAM-1 permet de calculer une valeur $K^*_d$ constante de dissociation apparente (VCAM-1) égale à $1{,}35.10^{-7}$ M, ce qui démontre la bonne interaction entre le peptide et la molécule VCAM-1.

Le test fonctionnel d'inhibition d'adhésion cellulaire par le peptide NNSKSHT (SEQ ID No3) (figure 2) permet

de calculer une valeur $IC_{50}$ (peptide) égale à $6,3.10^{-5}$ M, ce qui témoigne de l'interaction spécifique entre le peptide et VCAM-1.

**II.3.2 Produit de contraste Gd-DOTA-NNSKSHT**

**II.3.2.1. IRM vivo sur modèle d'hépatite induite par la Con-A chez la souris**

L'évolution du rapport signal/bruit (ΔSNR %) au cours du temps montre que l'injection i.v. de Gd-DOTA-NNSKSHT induit un plus fort rehaussement du signal IRM dans le modèle hépatite par rapport au foie à la souris saine. Les profils cinétiques des ΔSNR sont sensiblement identiques chez la souris atteinte d'hépatite et chez la souris saine après injection de 100 μmol Gd/kg de Gd-DOTA. Le signal enregistré après injection de Gd-DOTA-NNSKSHT chez la souris atteinte d'hépatite est plus fort dès les premières minutes d'acquisition et se maintient plus longtemps (au moins 70 minutes) que les signaux enregistrés chez la souris saine, ou ceux acquis après injection de Gd-DOTA (baisse du signal dès la 10$^{éme}$ minute pour les trois conditions).

(figure 3 : Suivi temporel du ciblage du foie chez des souris saines ou atteintes d'hépatite induite par Con-A (n = 6) après injection i.v. de 100 μmol Gd/kg de Gd-DOTA ou Gd-DOTA-NNSKSHT.)

Ces résultats démontrent que le greffage du peptide NNSKSHT (SEQ ID No3) sur Gd-DOTA permet de cibler spécifiquement l'hépatite induite par Con-A chez la souris, via le ciblage de VCAM-1.

**II.3.2..2. IRM vivo sur souris ApoE-/-**

Le suivi dynamique du signal IRM sur un modèle d'athérosclérose (souris apoE$^{-/-}$) montre que le greffage du peptide NNSKSHT (SEQ ID No3) sur Gd-DOTA permet de cibler spécifiquement la plaque d'athérome, via un ciblage probable de VCAM-1. En effet, l'injection i.v. du Gd-DOTA-NNSKSHT, à la dose 100 μmol Gd/kg, permet de détecter un rehaussement de signal supérieur à celui mesuré avec le produit non greffé Gd-DOTA au niveau de l'aorte abdominale. Cette différence est observée avec les deux séquences IRM (RARE et MSME) pour tous les temps d'acquisition (7 - 60 min post injection de produit de contraste).

Figure 4 : Suivi dynamique du ciblage de la plaque d'athérome chez des souris apoE$^{-/-}$ (n = 4-6)après injection de Gd-DOTA ou Gd-DOTA-NNSKSHT (100 μmol Gd/kg i.v.). Séquences RARE et MSME

Figure 5 : Ciblage de la plaque d'athérome chez une souris apoE$^{-/-}$ après injection de Gd-DOTA ou de Gd-DOTA-NNSKSHT (100 μmol Gd/kg i.v.). IRM de coupes sériées de l'aorte abdominale (zone cerclée) sur une longueur de 3,2 mm.

Séquence RARE, 27 minutes après injection de produit de contraste. Le rehaussement (blanc) de la zone cerclée correspond à la plaque d'athérome.

## Revendications

1. Composé de formule générale (I) suivante :

   Signal - Lien - Peptide (I)
   dans laquelle :

   - Signal représente une entité signal ;
   - Lien, présent ou non, représente une liaison chimique et
   - Peptide représente un peptide ciblant VCAM consistant au plus en 20 aminoacides, le peptide ciblant VCAM étant choisi parmi les peptides de formule suivante:

     a) X1-X2-X3-X4-X5-X6-X7-X8-X9 (1) (SEQ ID No1) avec :

     - X1 est absent ou choisi parmi la cystéine, la méthionine
     - X2 choisi parmi l'asparagine ou la glutamine
     - X3 choisi parmi l'asparagine ou la glutamine
     - X4 choisi parmi la sérine, la thréonine
     - X5 choisi parmi la lysine, l'arginine, l'histidine, l'ornithine
     - X6 choisi parmi la sérine, la thréonine
     - X7 choisi parmi l'histidine, l'arginine, la lysine
     - X8 choisi parmi la thréonine, la sérine
     - X9 est absent ou choisi parmi la cystéine, la méthionine de préférence le peptide CNNSKSHTC (SEQ ID No2) et le peptide NNSKSHT (SEQ ID No3)

   et les sels pharmaceutiquement acceptables des composés du a).

2. Composé selon la revendication 1 dans laquelle P est un peptide de formule CNNSKSHTC (SEQ ID No2) ou NNSKSHT (SEQ ID No3).

3. Composé selon la revendication 1 ou 2 dans laquelle Signal est un chélate couplé ou non à un métal paramagnétique M, M étant choisi avantageusement parmi Gd, Mn, Fe, Dy et Tm.

4. Composé selon la revendication 1 ou 2 dans laquelle Signal est un chélate couplé ou non à un radionucléide.

5. Composé selon la revendication 4 dans laquelle le radionucléide, est le Ga68 pour l'imagerie PET.

6. Composé selon les revendications 3 à 5 dans laquelle le chélate est choisi parmi : DOTA, DTPA, DO3A, HPDO3A, TRITA, TETA, BOPTA, NOTA, PCTA, DOTMA, AAZTA, HOPO et leurs dérivés.

7. Composé selon la revendication 1 à 6 dans laquelle Lien représente :

a) un groupe de formule : Q1-1-Q2,
dans laquelle Q1 et Q2 identiques ou différents représentent O, S, NH, $CO_2$, - NHCO, CONH, NHCONH, NHCSNH, $SO_2NH$- ou $NHSO_2$-,
et 1 représente un groupe alkyle, alcoxyalkyle, polyalkoxyalkylène, alcényle, alcynyle, alkyle interrompu par un ou plusieurs squarate, par un ou plusieurs aryles, avantageusement phényle, ou par un ou plusieurs groupes choisis parmi -NH-, -O-, -CO-, -NH(CO)-, -(CO)NH-, -O(CO)-, ou -(OC)O- ;,
b) un groupe $(CH_2)_n$, $(CH_2)_n$-CO-, -$(CH_2)_n$NH-CO- avec n = 2 à 10 , $(CH_2CH_2O)_q(CH_2)_r$-CO-, $(CH_2CH_2O)_q(CH_2)_r$-NH-CO- avec q = 1-10 et r=2-10,
$(CH_2)_n$-CONH-, $(CH_2)_n$-CONH-PEG, $(CH_2)_n$-NH-,

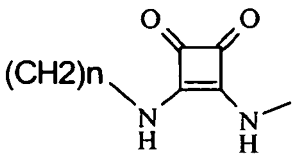

,

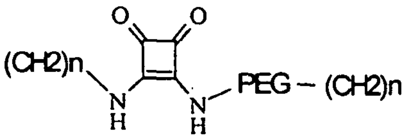

,

avec n=1 à 5 et avantageusement n=4 ou 5, HOOC-$CH_2$-O-$(CH_2)_2$-O-$(CH_2)_2$-O-$CH_2$-COOH ; HOOC-$(CH_2)_2$-$CO_2$-$(CH_2)_2$-OCO-$(CH_2)_2$-COOH ; HOOC-CH(OH)-CH(OH)-COOH; HOOC-$(CH_2)_n$-COOH; $NH_2$-$(CH_2)_n$-$NH_2$, avec n=1-20; $NH_2$-$(CH_2)_n$-$CO_2H$ ; ou $NH_2$-$CH_2$-$(CH_2$-O-$CH_2)_n$-$CO_2H$ avec n=1 à 10.

8. Composé selon la revendication 1 ou 2 dans laquelle Signal représente une nanoparticule superparamagnétique revêtue d'une couche organique, de préférence ayant un noyau en oxyde de fer, la couche organique étant avantageusement une couche gem-bisphosphonate.

9. Composé selon la revendication 1 ou 2 dans laquelle Signal représente un marqueur pour imagerie optique.

10. Composé selon la revendication 1 ou 2 dans laquelle Signal représente une nanoparticule lipidique comprenant au moins un chélate, avantageusement le chélate est tel que défini dans la revendication 6.

11. Composition pour imagerie médicale comprenant au moins un composé de formule générale (I) selon l'une des revendications 1 à 10 et un excipient pharmaceutiquement acceptable, avantageusement destinée à l'administration par voie parentérale.

12. Procédé de préparation de la composition selon la revendication 11 comprenant l'addition d'un composé de formule générale (I) selon l'une des revendications 1 à 10 à un milieu injectable comprenant l'excipient pharmaceutiquement acceptable.

13. Utilisation d'un composé (I) selon l'une des revendications 1 à 10 pour la préparation d'une composition de diagnostique de maladies cardiovasculaires, avantageusement choisie parmi : une maladie associée à des plaques d'athérome, en particulier des plaques vulnérables, une maladie coronarienne et/ou d'un risque d'accident ischémique, avantageusement choisi parmi : l'infarctus du myocarde, un accident vasculaire cérébral, une embolie rénale, une ischémie aiguë d'un membre, et une rupture d'anévrysme aortique.

14. Utilisation d'un composé (I) selon l'une des revendications 1 à 10 pour la préparation d'une composition de diagnostique destinée au suivi d'un traitement thérapeutique de maladies maladie cardiovasculaire et/ou d'un risque d'accident ischémique.

**15.** Utilisation d'un Peptide défini en revendications 1 ou 2 pour la préparation d'un médicament destiné au traitement thérapeutique ou préventif d'une maladie cardiovasculaire, le cas échéant en association avec un autre traitement thérapeutique ou préventif d'une maladie cardiovasculaire.

**Claims**

**1.** Compound of general formula (I) below:

Signal - Linker - Peptide (I)
in which:

- Signal represents a signal entity;
- Linker, which may or may not be present, represents a chemical bond, and
- Peptide represents a VCAM- targeting peptide consisting at most of 20 amino acids, the VCAM- targeting peptide being chosen from the peptides of formula below:

a) X1-X2-X3-X4-X5-X6-X7-X8-X9 (1) (SEQ ID No. 1) where:

- X1 is absent or chosen from cysteine and methionine
- X2 chosen from asparagine and glutamine
- X3 chosen from asparagine and glutamine
- X4 chosen from serine and threonine
- X5 chosen from lysine, arginine, histidine and ornithine
- X6 chosen from serine and threonine
- X7 chosen from histidine, arginine and lysine
- X8 chosen from threonine and serine
- X9 is absent or chosen from cysteine and methionine

preferably, the peptide CNNSKSHTC (SEQ ID No. 2) and the peptide NNSKSHT (SEQ ID No. 3), and the pharmaceutically acceptable salts of these compounds of a).

**2.** Compound according to Claim 1, in which P is a peptide of formula CNNSKSHTC (SEQ ID No. 2) or NNSKSHT (SEQ ID No. 3).

**3.** Compound according to Claim 1 or 2, in which Signal is a chelate which may or may not be coupled to a paramagnetic metal M, M being chosen advantageously from Gd, Mn, Fe, Dy and Tm.

**4.** Compound according to Claim 1 or 2, in which Signal is a chelate which may or may not be coupled to a radionucleide.

**5.** Compound according to Claim 4, in which the radionucleide is Ga68 for PET imaging.

**6.** Compound according to Claims 3 to 5, in which the chelate is chosen from: DOTA, DTPA, D03A, HPD03A, TRITA, TETA, BOPTA, NOTA, PCTA, DOTMA, AAZTA, HOPO and their derivatives.

**7.** Compound according to Claims 1 to 6, in which Linker represents:

a) a group of formula: Q1-1-Q2,
in which Q1 and Q2, which may be identical or different, represent O, S, NH, $CO_2$, -NHCO, CONH, NHCONH, NHCSNH, $SO_2NH$- or $NHSO_2$ - ,
and 1 represents an alkyl group, alkoxyalkyl group, polyalkoxyalkylene group, alkenyl group, alkynyl group, alkyl group interrupted with one or more squarates, with one or more aryls, advantageously phenyl, or with one or more groups chosen from -NH-, -O-, -CO-, -NH(CO)-, -(CO)NH-, -O(CO)- or -(OC)O-;
b) a $(CH_2)_n$, $(CH_2)_n$-CO-, -$(CH_2)_n$NH-CO-, where n = 2 to 10, $(CH_2CH_2O)_q(CH_2)_r$-CO-, $(CH_2CH_2O)_q(CH_2)_r$-NH-CO-, where q = 1-10 and r = 2-10, $(CH_2)_n$-CONH-, $(CH_2)_n$-CONH-PEG, $(CH_2)_n$-NH-,

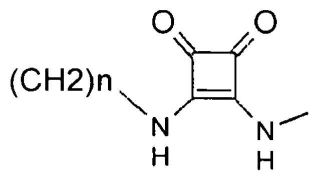,
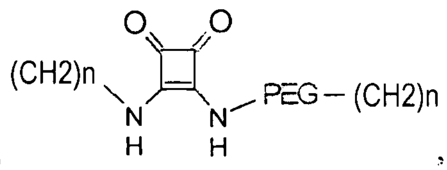,

where n = 1 to 5 and advantageously n = 4 or 5, $HOOC-CH_2-O-(CH_2)_2-O-(CH_2)_2-O-CH_2-COOH$; $HOOC-(CH_2)_2-CO_2-(CH_2)_2-OCO-(CH_2)_2-COOH$; HOOC-CH(OH)-CH(OH)-COOH; $HOOC-(CH_2)_n-COOH$; $NH_2-(CH_2)_n-NH_2$, where n = 1-20; $NH_2-(CH_2)_n-CO_2H$; or $NH_2-CH-(CH_2-O-CH_2)_n-CO_2H$, where n = 1 to 10, group.

**8.** Compound according to Claim 1 or 2, in which Signal represents a superparamagnetic nanoparticle coated with an organic layer, preferably having an iron oxide core, the organic layer advantageously being a gem-bisphosphonate layer.

**9.** Compound according to Claim 1 or 2, in which Signal represents a label for optical imaging.

**10.** Compound according to Claim 1 or 2, in which Signal represents a lipid nanoparticle comprising at least one chelate, advantageously the chelate is as defined in Claim 6.

**11.** Composition for medical imaging, comprising at least one compound of general formula (I) according to one of Claims 1 to 10 and a pharmaceutically acceptable excipient, advantageously for parenteral administration.

**12.** Method for preparing the composition according to Claim 11, comprising the addition of a compound of general formula (I) according to one of Claims 1 to 10 to an injectable medium comprising the pharmaceutically acceptable excipient.

**13.** Use of a compound (I) according to one of Claims 1 to 10, for the preparation of a composition for diagnosing cardiovascular diseases advantageously chosen from: a disease associated with atheroma plaques, in particular vulnerable plaques, and a coronary artery disease, and/or a risk of ischemic attack, advantageously chosen from: myocardial infarction, a cerebral stroke, a renal embolism, acute limb ischemia and a ruptured aortic aneurysm.

**14.** Use of a compound (I) according to one of Claims 1 to 10, for the preparation of a diagnostic composition for the use in monitoring a therapeutic treatment of a cardiovascular disease and/or a risk of ischemic attack.

**15.** Use of a Peptide defined in Claim 1 or 2, for the preparation of a medicament for use in the therapeutic or preventive treatment of a cardiovascular disease, where appropriate in combination with another therapeutic or preventive treatment of a cardiovascular disease.

**Patentansprüche**

**1.** Verbindung der folgenden allgemeinen Formel (I):

Signal - Bindung - Peptid (I)
worin:

- Signal für eine Signaleinheit steht;
- Bindung, die vorhanden ist oder nicht, für eine chemische Bindung steht und
- Peptid für ein VCAM- ähnliches Peptid steht, das aus höchstens 20 Aminosäuren besteht, wobei das VCAM- ähnliche Peptid aus den Peptiden der folgenden Formel ausgewählt ist:

a) X1-X2-X3-X4-X5-X6-X7-X8-X9 (I) (SEQ ID NR: 1); wobei:

- X1 fehlt oder aus Cystein, Methionin ausgewählt ist,

- X2 aus Asparagin oder Glutamin ausgewählt ist,
- X3 aus Asparagin oder Glutamin ausgewählt ist,
- X4 aus Serin, Threonin ausgewählt ist,
- X5 aus Lysin, Arginin, Histidin, Ornithin ausgewählt ist,
- X6 aus Serin, Threonin ausgewählt ist,
- X7 aus Histidin, Arginin, Lysin ausgewählt ist,
- X8 aus Threonin, Serin ausgewählt ist,
- X9 fehlt oder aus Cystein, Methionin ausgewählt ist,

vorzugsweise das Peptid CNNSKSHTC (SEQ ID NR: 2) und das Peptid NNSKSHT (SEQ ID NR: 3) und den pharmazeutisch annehmbaren Salzen der Verbindungen unter a).

**2.** Verbindung nach Anspruch 1, worin P ein Peptid der Formel CNNSKSHTC (SEQ ID NR: 2) oder NNSKSHT (SEQ ID NR: 3) ist.

**3.** Verbindung nach Anspruch 1 oder 2, worin das Signal ein Chelat ist, das an ein paramagnetisches Metall M gekuppelt ist oder nicht, wobei M vorteilhafterweise aus Gd, Mn, Fe, Dy und Tm ausgewählt ist.

**4.** Verbindung nach Anspruch 1 oder 2, worin das Signal ein Chelat ist, das an ein Radionuklid gekuppelt ist oder nicht.

**5.** Verbindung nach Anspruch 4, wobei es sich bei dem Radionuklid um Ga68 für die PET-Bildgebung handelt.

**6.** Verbindung nach den Ansprüchen 3 bis 5, wobei das Chelat aus folgenden ausgewählt ist: DOTA, DTPA, D03A, HPDO3A, TRITA, TETA, BOPTA, NOTA, PCTA, DOTMA, AAZTA, HOPO und ihren Derivaten.

**7.** Verbindung nach Anspruch 1 bis 6, worin Bindung für folgendes steht:

a) eine Gruppe der Formel: Q1-1-Q2,
worin Q1 und Q2, die gleich oder verschieden sind, für O, S, NH, $CO_2$, -NHCO, CONH, NHCONH, NHCSNH, $SO_2NH$-oder $NHSO_2$- stehen
und 1 für eine Alkyl-, Alkoxyalkyl-, Polyalkoxyalkylen-, Alkenyl-, Alkinylgrupe, eine Alkylgruppe, die durch ein oder mehrere Squarate, durch ein oder mehrere Aryle, vorzugsweise Phenyl, oder durch eine oder mehrere Gruppen, die aus -NH-, -O-, -CO-, - NH (CO) -, - (CO) NH-, -O (CO) - oder - (OC)O- ausgewählt sind, unterbrochen ist, steht;
b) eine Gruppe $(CH_2)_n$, $(CH_2)_n$-CO-, $(CH_2)_n$NH-CO- mit n = 2 bis 10, $(CH_2CH_2O)_q(CH_2)_r$-CO-, $(CH_2CH_2O)_q(CH_2)_r$-NH-CO- mit q = 1-10 und r = 2-10, $(CH_2)_n$-CONH-, $(CH_2)_n$-CONH-PEG,
$(CH_2)_n$-NH-,

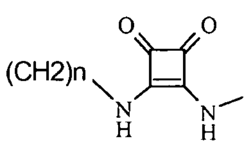
,
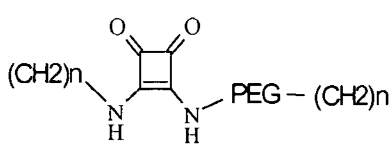

mit n = 1 bis 5 und vorteilhafterweise n = 4 oder 5, HOOC-$CH_2$-O-$(CH_2)_2$-O-$(CH_2)_2$-O-$CH_2$-COOH, HOOC-$(CH_2)_2$-$CO_2$-$(CH_2)_2$-OCO-$(CH_2)_2$-COOH, HOOC-CH(OH)-CH(OH)-COOH, HOOC- $(CH_2)_n$-COOH, $NH_2$-$(CH_2)_n$-$NH_2$ mit n = 1-20, $NH_2$-$(CH_2)_n$-$CO_2H$ oder $NH_2$-$CH_2$-$(CH_2$-O-$CH_2)_n$-$CO_2H$ mit n = 1 bis 10.

**8.** Verbindung nach Anspruch 1 oder 2, wobei Signal für ein superparamagnetisches Nanopartikel steht, das mit einer organischen Schicht beschichtet ist und vorzugsweise einen Eisenkern hat, wobei die organische Schicht vorzugsweise eine gem-Bisphosphonat-Schicht ist.

**9.** Verbindung nach Anspruch 1 oder 2, wobei Signal für einen Marker für die optische Bildgebung steht.

**10.** Verbindung nach Anspruch 1 oder 2, wobei Signal für ein Lipid-Nanopartikel steht, das mindestens ein Chelat umfasst, wobei das Chelat vorzugsweise wie im Anspruch 6 definiert ist.

**11.** Zusammensetzung für die medizinische Bildgebung, die mindestens eine Verbindung der allgemeinen Formel (I) nach einem der Ansprüche 1 bis 10 und einen pharmazeutisch annehmbaren Excipienten umfasst und die vorzugsweise für die Verabreichung auf parenteralem Weg bestimmt ist.

**12.** Verfahren zur Herstellung der Zusammensetzung nach Anspruch 11, das die Zugabe einer Verbindung der Formel (I) nach einem der Ansprüche 1 bis 10 zu einem injizierbaren Medium umfasst, das einen pharmazeutisch annehmbaren Excipienten umfasst.

**13.** Verwendung einer Verbindung (I) nach einem der Ansprüche 1 bis 10 zur Herstellung einer diagnostischen Zusammensetzung für kardiovaskuläre Krankheiten, die vorteilhafterweise aus folgenden ausgewählt sind: einer Krankheit, die mit Atheromplaques, insbesondere vulnerablen Plaques, einhergeht, einer koronaren Erkrankung, und/oder ein Risiko einer ischämischen Attacke, die vorteilhafterweise aus folgenden ausgewählt ist: Myokardinfarkt, Hirnschlag, Nierenembolie, akuter Ischämie eines Körperglieds und Aortenaneurysmaruptur.

**14.** Verwendung einer Verbindung (I) nach einem der Ansprüche 1 bis 10 zur Herstellung einer diagnostischen Zusammensetzung, die für die Nachverfolgung einer therapeutischen Behandlung der Krankheiten kardiovaskuläre Erkrankung und/oder eines Risikos für eine ischämische Attacke bestimmt ist.

**15.** Verwendung eines Peptids nach Anspruch 1 oder 2 zur Herstellung eines Medikaments, das für die therapeutische oder präventative Behandlung einer kardiovaskulären Erkrankung, gegebenenfalls in Verbindung mit einer anderen therapeutischen oder präventativen Behandlung einer kardiovaskulären Erkrankung, bestimmt ist.

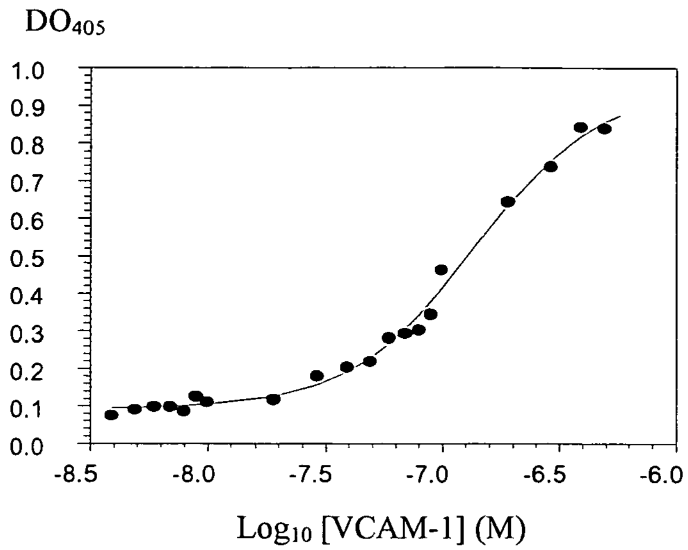


**FIGURE 1**

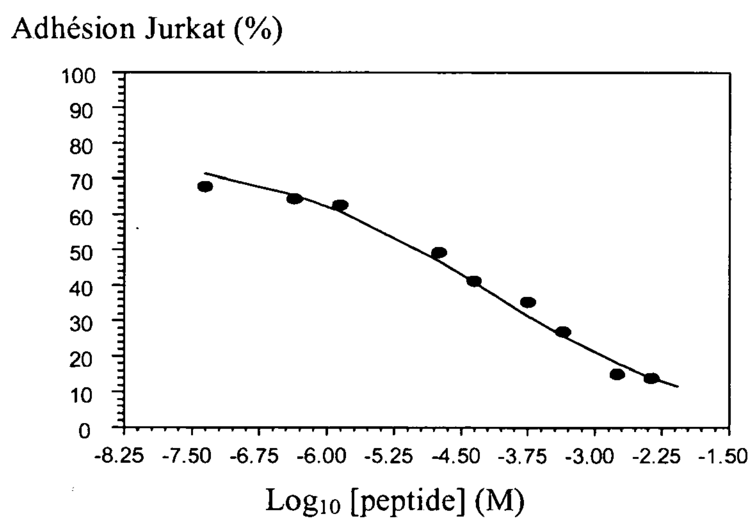


**FIGURE 2**

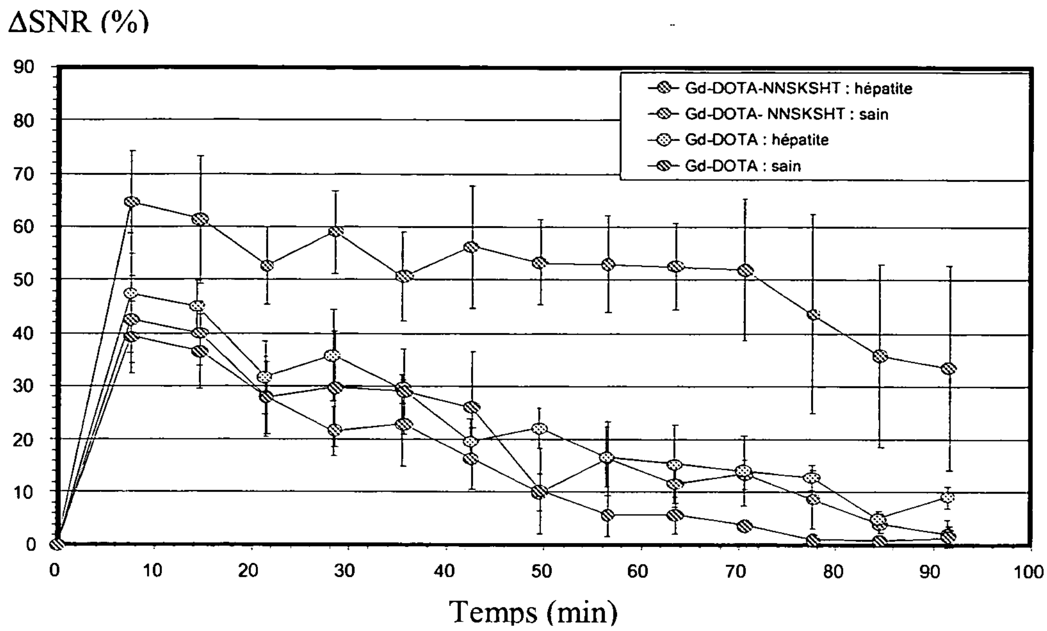


FIGURE 3

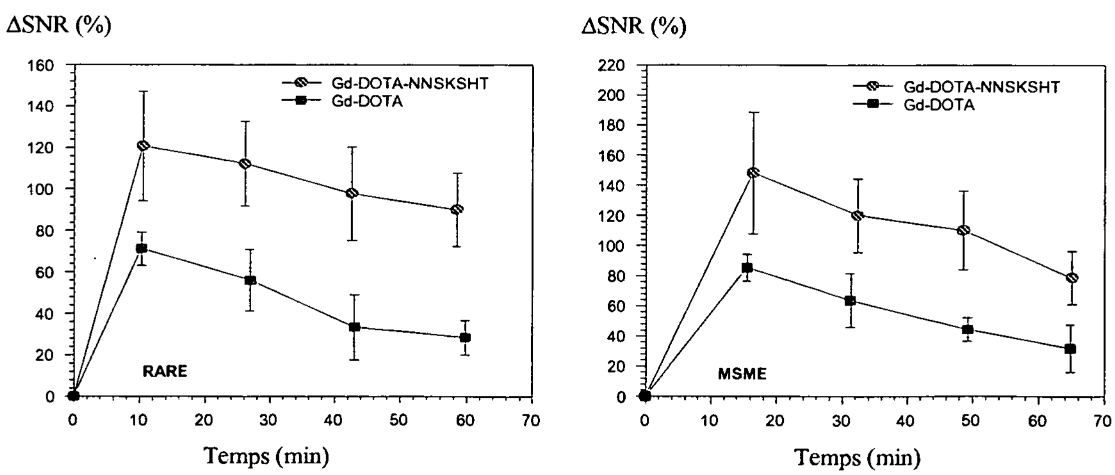


FIGURE 4

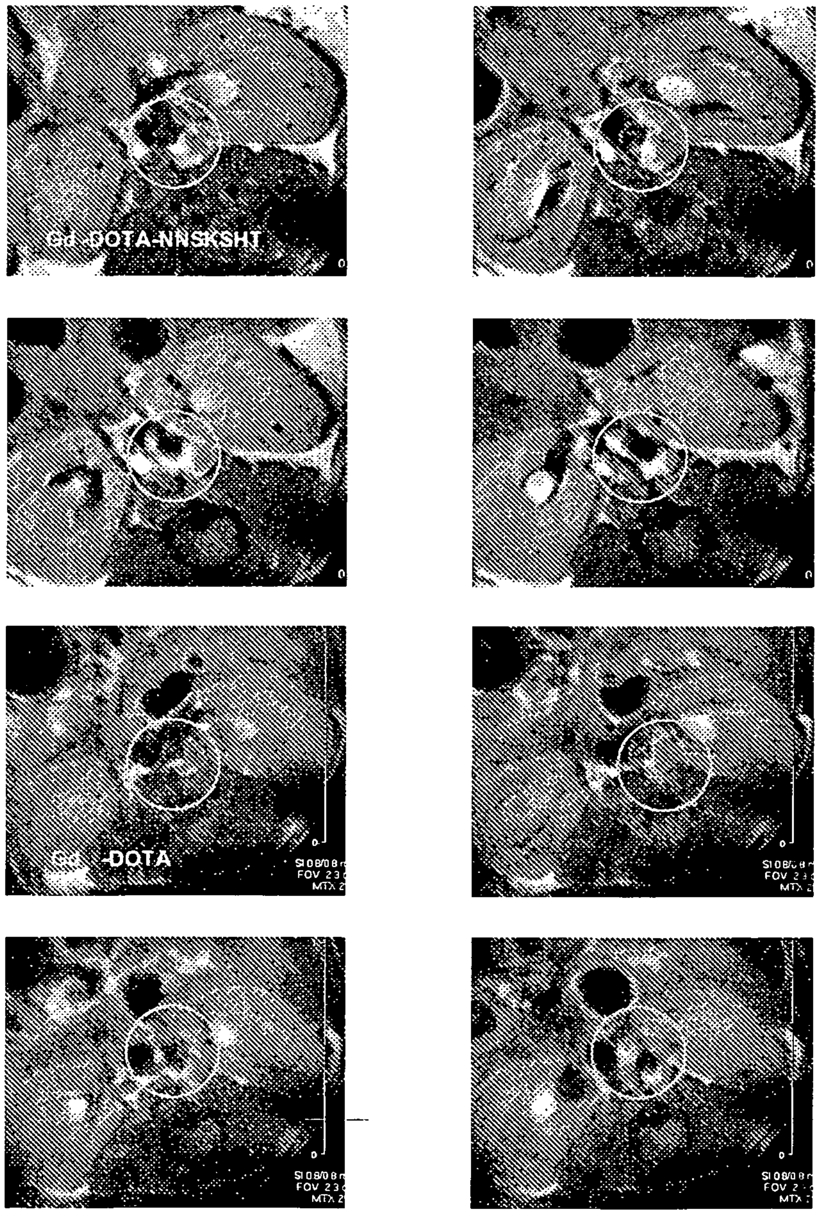


FIGURE 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03038130 A
- US 6110457 A
- US 2005100963 A
- WO 200600273 A
- US 200600118830 A
- DE 102004062258
- WO 0160416 A
- WO 03011115 A
- EP 661279 A
- US 5919432 A
- US 6440956 B
- US 20060018830 A
- US 6264914 B
- US 6537520 B
- WO 02085908 A
- WO 2004058275 A
- WO 03062198 A
- US 5958371 A
- US 5080885 A
- US 6403056 B
- US 6045821 A
- WO 9004943 A
- WO 2006100305 A
- US 6676963 B
- US 5989520 A
- US 20040248856 A
- US 6010682 A
- WO 2006032705 A
- US 20020090342 A
- US 20020098149 A
- WO 02055111 A
- WO 0226776 A
- US 20050100963 A
- WO 2006031885 A
- WO 200603788 A
- US 2004253181 A
- FR 0754087


### Littérature non-brevet citée dans la description


- **R. Pecora.** *J. of Nano. Res.,* 2000, vol. 2, 123-131
- **Burtea C ; Laurent S ; Vander Elst L ; Toubeau G ; Muller RN.** *J. Immunol.,* 2006, vol. 166, 1300-1307

LISTE DE SEQUENCES

<110> GUERBET

<120> Composés pour le diagnostique de maladies liées à l'expression de VCAM
PORT Marc
ROUSSEAUX Olivier
MULLER Robert
BURTEA Carmen

<130> D25382

<150> FR0754087
<151> 2007-03-28

<160> 5

<170> PatentIn version 3.3

<210> 1
<211> 9

<212> PRT
<213> Séquence artificielle

<220>
<223> marqueur de VCAM

<220>
<221> VARIANT
<222> (1)..(1)
<223> Xaa représente la cystéine ou la méthionine

<220>
<221> VARIANT
<222> (2)..(3)
<223> Xaa représente l'asparagine ou la glutamine

<220>
<221> VARIANT
<222> (4)..(4)
<223> Xaa représente la sérine ou la thréonine

<220>
<221> VARIANT
<222> (5)..(5)
<223> Xaa représente la lysine ou l'arginine ou l'histidine ou l'ornithine

<220>
<221> VARIANT
<222> (6)..(6)
<223> Xaa représente la sérine ou la thréonine

<220>
<221> VARIANT
<222> (7)..(7)
<223> Xaa représente la l'histidine ou l'arginine ou la lysine

<220>
<221> VARIANT
<222> (8)..(8)
<223> Xaa représente la thréonine ou la sérine

<220>
<221> VARIANT
<222> (9)..(9)
<223> Xaa représente la cystéine ou la méthionine

<400> 1

```
Xaa Xaa Xaa Xaa Xaa Xaa Xaa Xaa Xaa
1               5
```

<210> 2
<211> 9
<212> PRT
<213> Séquence artificielle

<220>

<223> marqueur de VCAM

<400> 2

```
Cys Asn Asn Ser Lys Ser His Thr Cys
1               5
```

<210> 3
<211> 7
<212> PRT
<213> Séquence artificielle

<220>
<223> marqueur de VCAM

<400> 3

```
Asn Asn Ser Lys Ser His Thr
1               5
```

<210> 4
<211> 9
<212> PRT
<213> Séquence artificielle

<220>
<223> marqueur de VCAM

<220>
<221> VARIANT
<222> (1)..(2)
<223> Xaa représente la cystéine ou la méthionine

<220>
<221> VARIANT
<222> (3)..(3)
<223> Xaa représente la lysine ou l’arginine ou l’alanine

<220>
<221> VARIANT
<222> (4)..(4)
<223> Xaa représente la thréonine ou la sérine

<220>
<221> VARIANT
<222> (5)..(5)
<223> Xaa représente l’acide aspartique ou l’acide glutamique

<220>
<221> VARIANT
<222> (6)..(6)
<223> Xaa représente la thréonine ou la sérine

<220>
<221> VARIANT
<222> (7)..(7)

<223> Xaa représente l'arginine ou l'alanine ou la lysine

<220>
<221> VARIANT
<222> (8)..(8)
<223> Xaa représente la leucine ou l'isoleucine ou la valine

<220>
<221> VARIANT
<222> (9)..(9)
<223> Xaa représente la cystéine ou la méthionine

<400> 4

```
Xaa Xaa Xaa Xaa Xaa Xaa Xaa Xaa Xaa
1               5
```